(19) Europäisches Patentamt
European Patent Office
Office européen des brevets

(11) EP 0 602 638 B1

(12) EUROPÄISCHE PATENTSCHRIFT

(45) Veröffentlichungstag und Bekanntmachung des Hinweises auf die Patenterteilung:
**06.03.1996 Patentblatt 1996/10**

(21) Anmeldenummer: **93120311.1**

(22) Anmeldetag: **16.12.1993**

(51) Int Cl.[6]: **C07F 15/00**, B01J 31/22, C07C 245/22, C08G 77/38, C08L 83/05, C07C 245/24, C07C 255/65, C07F 7/08, B01J 31/24

(54) **Katalysatoren für Hydrosilylierungsreaktionen**

Catalysts for hydrosilylation reactions

Catalyseurs de réaction d'hydrosilylation

(84) Benannte Vertragsstaaten:
**AT BE DE ES FR GB IT NL SE**

(30) Priorität: **16.12.1992 DE 4242469**

(43) Veröffentlichungstag der Anmeldung:
**22.06.1994 Patentblatt 1994/25**

(73) Patentinhaber: **Wacker-Chemie GmbH**
**D-81737 München (DE)**

(72) Erfinder:
- **Dr. Jochen Dauth**
  **D-84489 Burghausen (DE)**
- **Peetz, Udo**
  **D-84489 Burghausen (DE)**
- **Dr. Deubzer, Bernward**
  **D-84489 Burghausen (DE)**

(56) Entgegenhaltungen:

- **CHEMICAL ABSTRACTS, vol. 111, no. 3, 17. Juli 1989, Columbus, Ohio, US; abstract no. 23725q, KAMEDA, T. 'DIHYDRO(1,3-DIPHENYLTRIAZENIDO )(BIS(1,2-DIPHENYLPHOSPHINO)BENZENE)R HODIU M' Seite 637 ;**
- **CHEMICAL ABSTRACTS, vol. 112, no. 22, 28. Mai 1990, Columbus, Ohio, US; abstract no. 200382u, KATO, K. ET AL. 'FUNCTIONAL DIOLEFIN POLYMERS AND RHODIUM COMPLEX CATALYSTS FOR THEIR SELECTIVE HYDROGENATION' Seite 90 ;**
- **CHEMICAL ABSTRACTS, vol. 102, no. 6, 11. Februar 1985, Columbus, Ohio, US; abstract no. 46319p, KAMEDA, N. ET AL. 'POLYMERIZATION OF VINYL MONOMERS WITH A DIHYDRORHODIUM COMPLEX-TETRAHYDROFURAN SYSTEM' Seite 5 ;**
- **JOURNAL OF ORGANOMETALLIC CHEMISTRY Bd. 139 , 1979 Seiten 349 - 354 TONIOLO, L. ET AL. 'REDUCTIVE ELIMINATION OF 1,3-DI-P-TOLYLTRIAZENE IN REACTIONS OF TRANS-(PT(PPH3)2H(P-CH3C6H4N-N=NC6H4 CH3-P) WITH CO, 2,6-ME2C6H3NC, PPH3, AND PHC*CPH'**
- **CHEMICAL ABSTRACTS, vol. 91, no. 20, 12. November 1979, Columbus, Ohio, US; abstract no. 167611r, TONIOLO, L. ET AL. 'REACTIVITY OF M(ARN-N=NAR)(CO)(PPH3)2 WITH ARYLDIAZONIUM SALTS (M = RHODIUM(I), IRIDIUM(I); AR = P-CH3C6H4, P-FC6H4)' Seite 688 ;**
- **CHEMICAL ABSTRACTS, vol. 83, no. 8, 25. August 1975, Columbus, Ohio, US; abstract no. 70786y, MAJUMDAR, A.K. ET AL. 'COORDINATION COMPLEXES OF TRANSITIONAL METALS WITH 1-PHENYL-3-.ALPHA.-PYRIDYLTRIAZENE' Seite 843 ;**
- **CHEMICAL ABSTRACTS, vol. 114, no. 6, 11. Februar 1991, Columbus, Ohio, US; abstract no. 54746p, LEE, S.W. 'TETRAZENE COMPLEXES OF TRANSITION METALS'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

- JOURNAL OF ORGANOMETALLIC CHEMISTRY Bd. 208 , 1981 Seiten C21 - C24 OVERBOSCH, P. ET AL. 'SYNTHESIS OF METAL-TETRAAZADIENE COMPLEXES VIA LIGAND TRANSFER; TWO ROUTES TO NICKEL- OR PLATINUM-TETRAAZADIENE COMPLEXES (M(AR2N4)(L)2)'
- SYNTHESIS 1982 Seiten 49 - 50 JULLIARD, M. ET AL. 'SYNTHESIS OF PLATINUM COMPLEXES WITH ANTITUMOUR ACTIVITY'
- CHEMICAL ABSTRACTS, vol. 106, no. 20, 18. Mai 1987, Columbus, Ohio, US; abstract no. 167625b, MOORE, D.S. ET AL. 'CATENATED NITROGEN LIGANDS PART 1. TRANSITION METAL DERIVATIVES OF TRIAZENES, TETRAZENES, TETRAZADIENES, AND PENTAZADIENES' Seite 767 ;
- JOURNAL OF ORGANOMETALLIC CHEMISTRY Bd. 459 , 1993 Seiten 359 - 364 DAUTH, J. ET AL. 'TETRAKIS(.ETA1.-1-PHENYL -3-N-HEXYLTRIAZENIDO)PLATIN(IV), DER ERSTE HOMOLEPTISCHE ARYLALKYLTRIAZENIDOPLATIN(IV)KOMPLEX'

## Beschreibung

Die Erfindung betrifft Übergangsmetallkatalysatoren. Die Erfindung betrifft weiterhin Aryl-Alkyl-Triazenido-Übergangsmetall-Komplexe und Verfahren zu deren Herstellung. Die Erfindung betrifft weiterhin vernetzbare Zusammensetzungen und ein Verfahren zur Umsetzung von Si-gebundenen Wasserstoffatomen aufweisenden Organosiliciumverbindungen mit aliphatischen Mehrfachbindungen aufweisenden organischen Verbindungen in Gegenwart von Katalysatoren.

Triazenido-, Tetrazenido-, Tetrazdienido- und Pentazdienido-Übergangsmetall-Komplexe sind aus D.S. Moore et al., Advances in Inorganic Chemistry and Radiochemistry, Vol. 30, Seite 1-68, 1986 bekannt.

Während Diaryl-Triazenido-Übergangsmetall-Komplexe bekannt sind, sind Aryl-alkyl-Triazenido-Übergangsmetall-Komplexe bisher nicht beschrieben. Lediglich in M. Julliard et al., Synthesis, Seite 49, 1982 ist ein Triazenido-Platinkomplex der Formel $PtZ_2(ArNNNMe)_2$ (Z = Cl, I; Ar = Arylrest; Me = Methylrest) mit Antitumoraktivität beschrieben.

Es ist bekannt, daß die Anlagerung von Si-gebundenem Wasserstoff an eine aliphatische Mehrfachbindung, die häufig als Hydrosilylierung bezeichnet wird, durch Katalysatoren, insbesondere Platinverbindungen, gefördert werden kann. Hierzu sei beispielsweise auf US-A 3,814,730 und US-A 2,823,218 verwiesen. Diese Katalysatoren zeichnen sich durch eine niedrige Aktivierungsenergie aus und müssen in additionsvernetzenden Systemen oftmals inhibiert werden.

Es bestand die Aufgabe Katalysatoren bereitzustellen, die eine hohe Aktivierungsenergie besitzen, keine Inhibierung in additionsvernetzenden Systemen benötigen, aber nach Aktivierung die Anlagerung von Si-gebundenem Wasserstoff an eine aliphatische Mehrfachbindung fördern. Die Aufgabe wird durch die Erfindung gelöst.

Gegenstand der Erfindung ist die Verwendung der Übergangsmetallkomplexe der allgemeinen Formel

$$MX_aY_b \qquad (I),$$

wobei M Pt, Pd, Rh, Ru, Os oder Ir ist,

X einen Triazen-, Tetrazen-, Tetrazdien- oder Pentazdien-Liganden ausgewählt aus der Gruppe von ANNNR, $ANNNRR^1$, $ANNNA^1$, $ANR^1NNNR^2A^1$, $ANNNNA^1$, $ANNNR^3NNA^1$ und $ANNNNNA^1$ bedeutet, wobei R einen einwertigen, gegebenenfalls substituierten Kohlenwasserstoffrest mit 1 bis 18 Kohlenstoffatomen je Rest oder einen Rest der Formel $-SiR^6_c(OR^6)_{3-c}$,

$R^1$, $R^2$ und $R^3$ gleich oder verschieden sind, ein Wasserstoffatom oder einen einwertigen gegebenenfalls substituierten Kohlenwasserstoffrest mit 1 bis 18 Kohlenstoffatomen je Rest,
und A und $A^1$ gleich oder verschieden sind, einen Rest der Formel

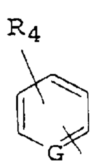 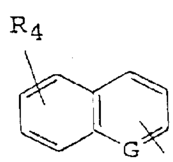 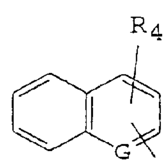 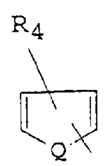 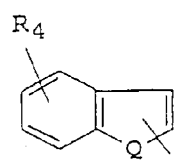 oder 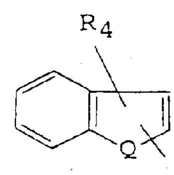

bedeuten, wobei G CH oder N und Q S, O oder NH ist,
$R^4$ einen einwertigen gegebenenfalls substituierten Kohlenwasserstoffrest mit 1 bis 12 Kohlenstoffatomen je Rest oder einen Rest der Formel -F, -Cl, -Br, -I, -H, $-NH_2$, $-NR^6_2$, $-NO_2$, -OH, $-OR^6$, -SH, -CN, -COOH, -COCl, $-CONH_2$, $-COR^6$, -CHO, $-SO_2NHR^6$, $-SO_3H$, $-SO_2Cl$ oder $-R^5-SiR^6_c(OR^6)_{3-c}$,
$R^5$ einen zweiwertigen Kohlenwasserstoffrest mit 1 bis 8 Kohlenstoffatomen je Rest,
$R^6$ gleich oder verschieden ist und einen Alkylrest mit 1 bis 8 Kohlenstoffatomen je Rest und
c 0, 1, 2 oder 3 bedeutet,
Y gleich oder verschieden ist und einen Liganden ausgewählt aus der Gruppe von Cl, Br, I, $NH_3$, $P(C_2H_5)_3$, $P(C_6H_5)_3$, H, CO, 1,5-Cyclooctadien, Pyridin, Bipyridin, Acetat, Acetylacetonat, Phenylnitril, Ethylendiamin, Acetonitril, 2,5-Norbornadien, Nitrat, Nitrit, $H_2O$, Benzol, Diphenylphosphinoethan und 1,3-Divinyl-1,1,3,3-tetramethyldisiloxan,
a 1, 2, 3 oder 4 und
b 0 oder eine ganze Zahl von 1 bis 6 bedeutet,
als die Anlagerung von Si-gebundenem Wasserstoff an aliphatische Mehrfachbindung fördernde Katalysatoren.

Gegenstand der Erfindung sind weiterhin Übergangsmetallkomplexe der allgemeinen Formel

$$M'X'_aY_b \qquad (II),$$

wobei M' Pt, Pd, Rh und Ru ist,
X' einen Triazen-Liganden der allgemeinen Formel
ANNNR' bedeutet, wobei R' einen einwertigen, gegebenenfalls substituierten Kohlenwasserstoffrest mit 1 bis 18 Kohlenstoffatomen je Rest, wobei aromatische Kohlenwasserstoffreste, bei denen der Aromat direkt am Stickstoffatom gebunden ist, ausgeschlossen sind, oder einen Rest der Formel $-SiR^6_c(OR^6)_{3-c}$ bedeutet, und
A, Y, a und b die oben dafür angegebene Bedeutung haben, mit der Maßgabe, daß Platin-Triazenido-Komplexe der Formel

$$PtZ_2[ANNN(CH_3)]_2,$$

wobei Z Cl oder I bedeutet und A die oben dafür angegebene Bedeutung hat, ausgeschlossen sind.

Gegenstand der Erfindung ist weiterhin ein Verfahren zur Herstellung der Übergangsmetallkomplexe, dadurch gekennzeichnet, daß Triazene der Formel
ANNNHR'

wobei A und R' die oben dafür angegebene Bedeutung haben, in Gegenwart von Basen mit Übergangsmetallverbindungen der Formel

$$M'Y_d$$

wobei M' und Y die oben dafür angegebene Bedeutung haben und d eine ganze Zahl von 1 bis 8 bedeutet, umgesetzt wird.

Triazenido-Komplexe von Übergangsmetallen - mit Ausnahme der Aryl-alkyl-triazenido-Komplexe von Übergangsmetallen -, Tetrazenido-Komplexe von Übergangsmetallen, Tetrazdienido-Komplexe von Übergangsmetallen und Pentazdienido-Komplexe von Übergangsmetallen sowie deren Struktur sind in der eingangs genannten Literaturstelle, D.S. Moore et al., Advances in Inorganic Chemistry and Radiochemistry, Vol. 30, Seite 1-68, 1986, beschrieben.

Beispiele für Reste R sind Alkylreste, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest; Hexylreste, wie der n-Hexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest, Dodecylreste, wie der n-Dodecylrest; und Octadecylreste, wie der n-Octadecylrest; Cycloalkylreste, wie der Cyclohexylrest; Alkenylreste, wie der Vinyl-, 1-Propenyl-, 1-Butenyl-, 2-Butenyl-, Allyl-, Isobutenyl-, 1-Pentenyl- und 2-Methyl-1-butenylrest; Alkinylreste, wie der Ethinyl-, Propargyl-, 1-Propinyl-und l-Butinylrest, und Aralkylreste, wie der Benzylrest und der α- und β-Phenylethylrest; wobei Alkylreste bevorzugt sind.

Beispiele für substituierte Kohlenwasserstoffreste R sind Halogenalkylreste, wie der 3,3,3-Trifluor-n-propylrest, der 2,2,2,2',2',2'-Hexafluorisopropylrest, der Heptafluorisopropylrest, der 3-Chlor-n-propylrest, 2-Ethylbromid und 3-Propylbromid; Hydroxyalkylreste, wie Reste der Formel $HOCH_2CH_2OCH_2CH_2-$, $HOCH_2CH_2-$ und $CH_3CH_2CH(OH)CH_2-$; Aminoalkylreste, wie der Aminomethyl- und Aminoethylrest; Carboxyalkylreste, wie Reste der Formel $-(CH_2)_7COOH$, $-(CH_2)_8COOH$ und $-CH_2COCH_2CH_2COOH$ sowie deren Ester und Amide $-(CH_2)_7COOCH_3$, $-(CH_2)_7COOC_2H_5$, $-(CH_2)_7CONH_2$, $-(CH_2)_8COOCH_3$, $-(CH_2)_8COOC_2H_5$, $-(CH_2)_8CONH_2$,
einen Rest der Formel $-CH(COOC_2H_5)_2$; und substituierte Aralkylreste, wie der substituierte Benzylrest und der substituierte α- und β-Phenylethylrest.

Beispiele für Kohlenwasserstoffreste R und substituierte Kohlenwasserstoffreste R gelten im vollen Umfang für Kohlenwasserstoffreste R' und substituierte Kohlenwasserstoffreste R'.

Beispiele für Reste $R^1$, $R^2$ und $R^3$ sind Alkylreste, wie der Methyl-, Ethyl-, n-Propyl-, iso-Propyl-, n-Butyl-, iso-Butyl-, tert.- Butyl-, n-Pentyl-, iso-Pentyl-, neo-Pentyl-, tert.-Pentylrest; Hexylreste, wie der n-Hexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest, Dodecylreste, wie der n-Dodecylrest; und Octadecylreste, wie der n-Octadecylrest; Alkenylreste, wie der Vinyl-, 1-Propenyl-, 1-Butenyl-, 2-Butenyl-, Allyl-, Isobutenyl-, 1-Pentenyl- und 2-Methyl-1-butenylrest; Alkinylreste, wie der Ethinyl-, Propargyl-, 1-Propinyl- und 1-Butinylrest; Arylreste, wie der Phenylrest; Alkarylreste, wie der o-, m- und p-Tolylrest, p-Ethylphenyl-, p-Butylphenyl und p-Hexylphenylrest; und Aralkylreste, wie der Benzylrest und der α- und β-Phenylethylrest.

Beispiele für substituierte Kohlenwasserstoffreste $R^1$, $R^2$ und $R^3$ sind, Halogenalkylreste, wie der 3,3,3-Trifluor-n-propylrest, der 2,2,2,2',2',2'-Hexafluorisopropylrest, der Heptafluorisopropylrest, der 3-Chlor-n-propylrest, 2-Ethylbromid und 3-Propylbromid; Halogenarylreste, wie der o-, m-, und p-Chlorphenylrest, o-, m- und p-Bromphenylrest; substituierte Arylreste, wie der 4-Cyanophenyl-, 4-Nitrophenyl- und 4-Methoxyphenylrest; Hydroxyalkylreste, wie Reste der Formel $HOCH_2CH_2OCH_2CH_2$-, $HOCH_2CH_2$-und $CH_3CH_2CH(OH)CH_2$-; Aminoalkylreste, wie der Aminomethyl- und Aminoethylrest; Carboxyalkylreste, wie Reste der Formel $-(CH_2)_7COOH$, $-(CH_2)_8COOH$ und $-CH_2COCH_2CH_2COOH$ sowie deren Ester und Amide $-(CH_2)_7COOCH_3$, $-(CH_2)_7COOC_2H_5$, $-(CH_2)_7CONH_2$, $-(CH_2)_8COOCH_3$, $-(CH_2)_8COOC_2H_5$, $-(CH_2)_8CONH_2$ und einen Rest der Formel $-CH(COOC_2H_5)_2$; Carboxyarylreste, wie der 4-Carboxyphenyl-, 3-Carboxyphenylrest und Reste der Formel $4-CH_3OCOC_6H_4$-, $4-C_2H_5OCOC_6H_4$- und $4-H_2NCOC_6H_4$-; und substituierte Aralkylreste, wie der substituierte Benzylrest und der substituierte $\alpha$- und $\beta$-Phenylethylrest.

Beispiele für Kohlenwasserstoffreste $R^4$ sind Alkylreste, wie der Methyl-, Ethyl-, n-Propyl-, iso-Propyl-, n-Butyl-, iso-Butyl-, tert.- Butyl-, n-Pentyl-, iso-Pentyl-, neo-Pentyl-, tert. -Pentylrest; Hexylreste, wie der n-Hexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest, Dodecylreste, wie der n-Dodecylrest; und Octadecylreste, wie der n-Octadecylrest; Alkenylreste, wie der Vinyl-, 1-Propenyl-, 1-Butenyl-, 2-Butenyl-, Allyl-, Isobutenyl-, 1-Pentenyl- und 2-Methyl-1-butenylrest; Alkinylreste, wie der Ethinyl-, Propargyl-, 1-Propinyl- und 1-Butinylrest; und Arylreste, wie der Phenylrest.

Beispiele für substituierte Kohlenwasserstoffreste $R^4$ sind Alkylalkoxyreste, wie der Methylmethoxy-, Ethylmethoxy-, Methylethoxy-, Ethylethoxy-, Methylisopropoxy-, Ethylisopropoxy-, Methylbutoxy- und Ethylbutoxyrest; Aryloxyreste, wie der Phenoxyrest; substituierte Arylreste, wie der 4-Bromphenyl-, 4-Cyanophenyl-, 4-Nitrophenyl- und 4-Methoxyphenylrest; Hydroxyalkylreste, wie Reste der Formel $HOCH_2CH_2OCH_2CH_2$-, $HOCH_2CH_2$-und $CH_3CH_2CH(OH)CH_2$-; Aminoalkylreste, wie der Aminomethyl- und Aminoethylrest; Carboxyalkylreste, wie Reste der Formel $-(CH_2)_7COOH$, $-(CH_2)_8COOH$ und $-CH_2COCH_2CH_2COOH$ sowie deren Ester und Amide $-(CH_2)_7COOCH_3$, $-(CH_2)_7COOC_2H_5$, $-(CH_2)_7CONH_2$, $-(CH_2)_8COOCH_3$, $-(CH_2)_8COOC_2H_5$, $-(CH_2)_8CONH_2$ und einen Rest der Formel $-CH(COOC_2H_5)_2$; Carboxyarylreste, wie der 4-Carboxyphenyl-, 3-Carboxyphenylrest und Reste der Formel $4-CH_3OCOC_6H_4$-, $4-C_2H_5OCOC_6H_4$- und $4-H_2NCOC_6H_4$-.

Beispiele für Kohlenwasserstoffreste $R^5$ sind lineare oder verzweigte Alkylenreste, wie der Methylen-, Ethylen-, Propylen-, 2-Methylpropylen- und Butylenrest.

Beispiele für Alkylreste $R^6$ sind der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest; Hexylreste, wie der n-Hexylrest; Heptylreste, wie der n-Heptylrest; und Octylreste, wie der n-Octylrest.

Die Reste $R^4$ sind Substituenten des Aromaten- und Heteroaromatenrestes A bzw. A' und können sich beispielsweise, wenn A bzw. A' ein aromatischer Sechsring, wie Phenylrest ist, in ortho-, meta- oder para-Stellung befinden.

Beispiele für Katalysatoren sind solche der Formel

$PtX_aY_b$ mit a = 2 oder 4 und b = 2, 1 oder 0
$PdX_aY_b$ mit a = 1 oder 2 und b = O oder 1
$RuX_aY_b$ mit a = 1, 2, 3 oder 4 und b = 0, 1, 2 oder 3
$RhX_aY_b$ mit a = 1, 2 oder 3 und b = 0, 1 oder 2
$OsX_aY_b$ mit a = 3 oder 4 und b = 0, 1, 2 oder 3
$IrX_aY_b$ mit a = 1, 2, 3 oder 4 und b = 0, 1, 2 oder 3,

wobei X und Y die oben dafür angegebene Bedeutung haben.

Bevorzugt als Katalysatoren sind die Triazenido-Komplexe, insbesondere mit dem Liganden ANNNR, und die Pentazdienido-Komplexe mit dem Liganden $ANNNNNA^1$.

Bevorzugte Beispiele für den Triazen-Liganden ANNNR sind solche der Formel

$C_6H_5NNN(CH_2)_xCH_3$
$p-NO_2-C_6H_4NNN(CH_2)_xCH_3$,
$p-CN-C_6H_4NNN(CH_2)_xCH_3$ und
$p-CH_3(CH_2)_x-C_6H_4NNN(CH_2)_xCH_3$.

wobei x 1, 3, 5, 7, 11 und 17, insbesondere 1, 5, 7 und 11 ist.

Bevorzugte Beispiele für die Liganden $ANNNNNA^1$ sind solche der Formel

$p-Br-C_6H_4NNNNNC_6H_4-Br-p$ und
$p-CH_3O-C_6H_4NNNNNC_6H_4-OCH_3-p$.

Bevorzugte Beispiele für Katalysatoren sind solche der Formel

$Pt[C_6H_5NNN(CH_2)_xCH_3]_4$
$Pt[p\text{-}NO_2\text{-}C_6H_4NNN(CH_2)_xCH_3]_4$
$Pt[p\text{-}CN\text{-}C_6H_4NNN(CH_2)_xCH_3]_4$
1,5-Cyclooctadien·$Pt[C_6H_5NNN(CH_2)_xCH_3]_2$
1,5-Cyclooctadien·$Pt[p\text{-}NO_2\text{-}C_6H_4NNN(CH_2)_xCH_3]_2$
1,5-Cyclooctadien·$Pt[p\text{-}CN\text{-}C_6H_4NNN(CH_2)_xCH_3]_2$
$Pd[C_6H_5NNN(CH_2)_xCH_3]_2$
$Pd[p\text{-}NO_2\text{-}C_6H_4NNN(CH_2)_xCH_3]_2$
$Pd[p\text{-}CN\text{-}C_6H_4NNN(CH_2)_xCH_3]_2$
$(C_6H_5P)_3Ru[C_6H_5NNN(CH_2)_xCH_3]_2$
$(C_6H_5P)_3Ru[p\text{-}NO_2\text{-}C_6H_4NNN(CH_2)_xCH_3]_2$
$(C_6H_5P)_3Ru[p\text{-}CN\text{-}C_6H_4NNN(CH_2)_xCH_3]_2$
$(C_6H_5P)_3Rh[C_6H_5NNN(CH_2)_xCH_3]$
$(C_6H_5P)_3Rh[p\text{-}NO_2\text{-}C_6H_4NNN(CH_2)_xCH_3]$
$(C_6H_5P)_3Rh[p\text{-}CN\text{-}C_6H_4NNN(CH_2)_xCH_3]$
$Pt[p\text{-}Br\text{-}C_6H_4NNNNNC_6H_4\text{-}Br\text{-}p]_4$
$Pt[p\text{-}CH_3O\text{-}C_6H_4NNNNNC_6H_4\text{-}OCH_3\text{-}p]_4$
1,5-Cyclooctadien·$Pt[p\text{-}Br\text{-}C_6H_4NNNNNC_6H_4\text{-}Br\text{-}p]_2$
1,5-Cyclooctadien·$Pt[p\text{-}CH_3O\text{-}C_6H_4NNNNNC_6H_4\text{-}OCH_3\text{-}p]_2$
$Pt[p\text{-}CH_3(CH_2)_x\text{-}C_6H_4NNN(CH_2)_xCH_3]_2$,

wobei x 1, 3, 5, 7, 11 und 17, insbesondere 1, 5, 7 und 11 ist, und die oben genannten Triazenido-Komplexe des Platins besonders bevorzugt sind.

Die Herstellung der Triazenido-Komplexe von Übergangsmetallen - mit Ausnahme der Aryl-alkyl-triazenido-Komplexe von Übergangsmetallen -, der Tetrazenido-Komplexe von Übergangsmetallen, der Tetrazdienido-Komplexe von Übergangsmetallen und der Pentazdienido-Komplexe von Übergangsmetallen ist bekannt und in D.S. Moore et al., Advances in Inorganic Chemistry and Radiochemistry, Vol. 30, Seite 1-68, 1986 beschrieben.

Die Herstellung der Triazene, Tetrazene, Tetrazdiene und Pentazdiene ist bekannt und in T.P. Ahern et al., Can. J. Chem. 55, 1701 (1977) und M.A. Kelly et al., J. Chem. Soc., Perkin Trans. II, 1649 (1982) beschrieben.

Beispiele für Triazene der Formel ANNNHR', die bei der Herstellung der Aryl-alkyl-Triazenido-Komplexe der Formel $M'X'_aY_b$ eingesetzt werden, sind

$C_6H_5NNNH(CH_2)_xCH_3$
$p\text{-}NO_2\text{-}C_6H_4NNNH(CH_2)_xCH_3$
$p\text{-}CN\text{-}C_6H_4NNNH(CH_2)_xCH_3$
$p\text{-}CH_3\text{-}C_6H_4NNNH(CH_2)_xCH_3$
$p\text{-}CH_3(CH_2)_x\text{-}C_6H_4NNNH(CH_2)_xCH_3$
$p\text{-}H_3COCO\text{-}C_6H_4NNNH(CH_2)_xCH_3$
$p\text{-}CH_3NHCO\text{-}C_6H_4NNNH(CH_2)_xCH_3$
$p\text{-}CH_3O\text{-}C_6H_4NNNH(CH_2)_xCH_3$
$p\text{-}(CH_3)_2N\text{-}C_6H_4NNN(CH_2)_xCH_3$,
wobei $C_6H_5NNNH(CH_2)_xCH_3$
$p\text{-}NO_2\text{-}C_6H_4NNNH(CH_2)_xCH_3$
$p\text{-}CN\text{-}C_6H_4NNNH(CH_2)_xCH_3$ und
$p\text{-}CH_3(CH_2)_x\text{-}C_6H_4NNNH(CH_2)_xCH_3$
(x ist 1, 3, 5, 7, 11 und 17, insbesondere 1, 5, 7 und 11) bevorzugte Beispiele sind.

Beispiele für Übergangsmetallverbindungen der Formel $M'Y_d$, die bei Herszellung der Aryl-alkyl-Triazenido-Komplexe der Formel $M'X'_aY_b$ eingesetzt werden, sind $PtCl_2$, $PtJ_2$ , $[(C_6H_5)_3P]_2PtCl_2$, $[(C_2H_5)_3P]_2PtCl_2$, $PtCl_4$, $Pt(H_2NCH_2CH_2NH_2)Cl_2$, $Pt(NH_3)_2Cl_2$, $PtBr_2$, $PtJ_2$, 1,5-Cyclooctadien·$PtCl_2$, $Pd(CH_3CN)_2Cl_2$, $PdBr_2$, 1,5-Cyclooctadien·$PdCl_2$, $[(C_6H_5)_3P]_2PdCl_2$, $PdCl_2$, $RuCl_3$, $Ru(NH_3)_6Cl_2$, $[(C_6H_5)_3P]_3RuCl_2$, $RhCl_3$, $RhBr_3$, $[(C_6H_5)_3P]_3RhCl$, (1,5-Cyclooctadien)$_2$Pt, 1,3-Divinyl-1,1,3,3-tetramethyldisiloxanplatinkomplex (z.B. $Pt_2$[1,3-divinyl-1,1,3,3-tetramethyldisiloxan]$_3$), Pd[bis-(1,2-diphenyl-phosphinoethan)], Hexarhodiumhexadecacarbonyl und Trirutheniumdodecacarbonyl, wobei $PtCl_4$

1,5-Cyclooctadien·$PtCl_2$
$PtJ_2$
1,3-Divinyl-1,1,3,3-tetramethyldisiloxanplatinkomplex

$PdCl_2$
$[(C_6H_5)_3P]_3RuCl_2$ und
$[(C_6H_5)_3P]_3RhCl$ bevorzugt sind.

Beispiele für Basen, die bei Herstellung der Aryl-alkyl-Triazenido-Komplexe der Formel $M'X'_aY_b$ eingesetzt werden, sind n-Butyllithium, Triethylamin, Piperidin, Pyridin, $NaOCH_3$ und $NaNH_2$, wobei n-Butyllithium und Triethylamin bevorzugt sind.

Das Verfahren zur Herstellung der Aryl-alkyl-Triazenido-Komplexe $M'X'_aY_b$ wird vorzugsweise in Gegenwart von organischen Lösungsmitteln, wie n-Hexan, Toluol, Methylenchlorid, Chloroform, Aceton oder Tetrahydrofuran durchgeführt, kann aber auch in Gegenwart eines Gemisches aus Wasser und organischem Lösungsmittel, wie Methanol, Ethanol, Isopropanol oder Tetrahydrofuran durchgeführt werden.

Das Verfahren zur Herstellung der Aryl-alkyl-Triazenido-Komplexe $M'X'_aY_b$ wird vorzugsweise bei Temperaturen von 0°C bis 50°C beim Druck der umgebenden Atmosphäre und unter Lichtausschluß durchgeführt. Das organische Lösungsmittel bzw. das Gemisch aus organischem Lösungsmittel und Wasser wird vorzugsweise nach der Umsetzung entfernt.

Die Erfindung betrifft weiterhin ein Verfahren zur Aktivierung der erfindungsgemäßen Katalysatoren der Formel (II) durch Erhitzen bei Temperaturen von 50°C bis 250°C und/oder durch Bestrahlen mit Licht und/oder durch Zugabe von Brönsted-Säuren und/oder durch Zugabe von säurebildenden Agentien.

Bei den erfindungsgemäßen Katalysatoren hängt die Aktivierungstemperatur vom Liganden X und dem Übergangsmetallatom M des jeweiligen Komplexes ab. Als Licht mit dem die erfindungsgemäßen Katalysatoren aktiviert werden können, ist Ultraviolettlicht bevorzugt. Im Handel gibt es eine Vielzahl von Lampen, die Ultraviolettlicht im Bereich von 200 bis 400 nm aussenden. Die Aktivierung der erfindungsgemäßen Katalysatoren kann durch Erhitzen bei Temperaturen von 50°C bis 250°C und zusätzlich durch Bestrahlen mit Licht, bevorzugt Ultraviolettlicht, erfolgen. Beispiele für Brönsted-Säuren sind Essigsäure, $HNO_3$, $H_2SO_4$ und HCl. Als säurebildende Agentien werden vorzugsweise Jodonium- oder Sulfoniumsalze verwendet. Bevorzugt werden solche Jodonium- oder Sulfoniumsalze, wie in den deutschen Anmeldungen P 41 42 327.5 und P 42 19 376.1 beschrieben, verwendet. Beispiele für Jodonium- und Sulfoniumsalze sind solche der Formel

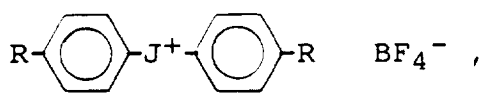

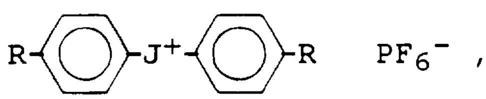

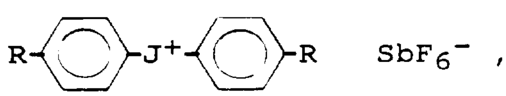

wobei R einen $C_1$-$C_{18}$-Kohlenwasserstoffrest, wie n-Dodecylrest, bedeutet,

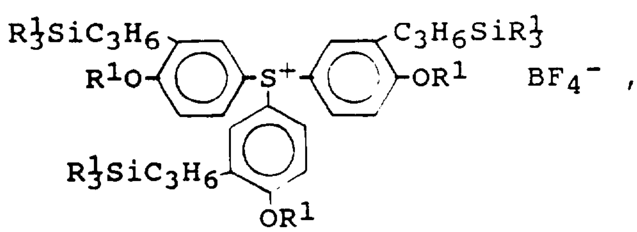

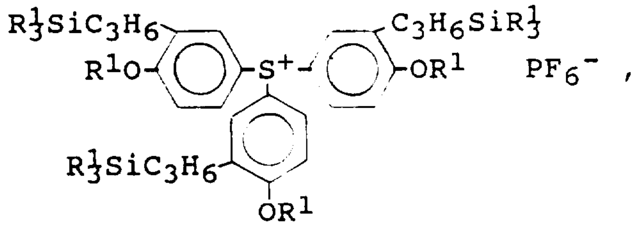

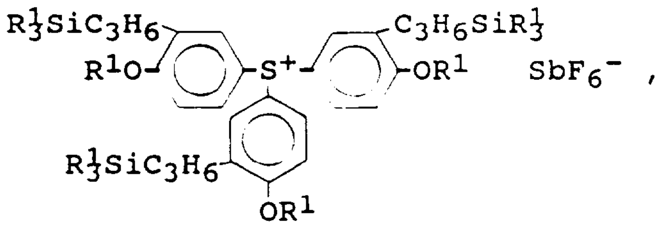

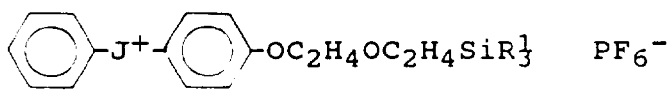

und

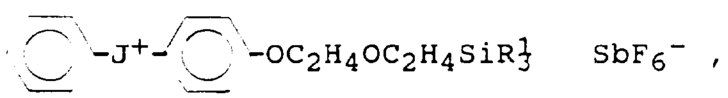

wobei $R^1$ einen $C_1$-$C_4$-Kohlenwasserstoffrest, wie n-Butyl-, Methyl-, Ethyl- oder Propylrest bedeutet.

Die Jodonium- und Sulfoniumsalze eliminieren bei Bestrahlung mit Licht, bevorzugt Ultraviolettlicht, starke Säuren.

Die erfindungsgemäßen Katalysatoren können in allen vernetzbaren Organopolysiloxanzusammensetzungen verwendet werden, in denen auch bisher Katalysatoren verwendet werden konnten, die die Anlagerung von Si-gebundenen Wasserstoff an aliphatische Mehrfachbindung fördern.

Gegenstand der Erfindung sind daher vernetzbare Organopolysiloxanzusammensetzungen enthaltend

(1) Organopolysiloxane, die Reste mit aliphatischen Kohlenstoff-Kohlenstoff-Mehrfachbindungen aufweisen,
(2) Organopolysiloxane mit Si-gebundenen Wasserstoffatomen oder anstelle von (1) und (2)
(3) Organopolysiloxane, die Reste mit aliphatischen Kohlenstoff-Kohlenstoff-Mehrfachbindungen und Si-gebundene Wasserstoffatome aufweisen, und
(4) Katalysatoren gemäß Formel (I).

Unter Resten mit aliphatischen Kohlenstoff-Kohlenstoff-Mehrfachbindungen sind auch Reste mit cycloaliphatischen Kohlenstoff-Kohlenstoff-Mehrfachbindungen zu verstehen.

Als Organopolysiloxane (1), die Reste mit aliphatischen Kohlenstoff-Kohlenstoff-Mehrfachbindungen aufweisen, werden vorzugsweise lineare oder verzweigte Organopolysiloxane aus Einheiten der Formel

$$R^7_n R^8_m SiO_{\frac{4-n-m}{2}}$$

wobei $R^7$ einen einwertigen von aliphatischen Kohlenstoff-Kohlenstoff-Mehrfachbindungen freien Kohlenwasserstoffrest mit 1 bis 18 Kohlenstoffatomen je Rest und

$R^8$ einen einwertigen Kohlenwasserstoffrest mit aliphatischer Kohlenstoff-Kohlenstoff-Mehrfachbindung mit 2 bis 8 Kohlenstoffatomen je Rest bedeutet,
n 0, 1, 2 oder 3,
m 0, 1 oder 2
und die Summe n+m 0, 1, 2 oder 3 ist,

mit der Maßgabe, daß durchschnittlich mindestens 2 Reste $R^8$ je Molekül vorliegen, verwendet. Die Organopolysiloxane (1) besitzen vorzugsweise eine durchschnittliche Viskosität von 100 bis 10 000 mPa·s bei 25°C.

Beispiele für Kohlenwasserstoffreste $R^7$ sind Alkylreste, wie der Methyl-, Ethyl-, n-Propyl-, iso-Propyl-, n-Butyl-, iso-Butyl-, tert.- Butyl-, n-Pentyl-, iso-Pentyl-, neo-Pentyl-, tert. -Pentylrest; Hexylreste, wie der n-Hexylrest; Heptylreste, wie der n-Heptylrest; Octylreste, wie der n-Octylrest und iso-Octylreste, wie der 2,2,4-Trimethyl-pentylrest; Nonylreste, wie der n-Nonylrest; Decylreste, wie der n-Decylrest, Dodecylreste, wie der n-Dodecylrest; Octadecylreste, wie der n-Octadecylrest; Cycloalkylreste, wie Cyclopentyl-, Cyclohexyl-, Cycloheptylreste und Methylcyclohexylreste; Arylreste, wie der Phenyl-, Naphthyl-, Anthryl- und Phenanthrylrest; Alkarylreste, wie o-, m-, p-Tolylreste, Xylylreste und Ethylphenylreste; und Aralkylreste, wie der Benzylrest, der α- und der β-Phenylethylrest.

Beispiele für Reste $R^8$ sind Alkenylreste, wie der Vinyl-, 5-Hexenyl-, 1-Propenyl-, Allyl-, 1-Butenyl- und 1-Pentenylrest; und Alkinylreste, wie der Ethinyl-, Propargyl- und 1-Propinylrest.

Als Organopolysiloxane (2), die Si-gebundene Wasserstoffatome aufweisen, werden vorzugsweise lineare, cyclische oder verzweigte Organopolysiloxane aus Einheiten der Formel

$$R^7_eH_fSiO_{\frac{4-e-f}{2}}$$

wobei $R^7$ die oben dafür angegebene Bedeutung hat,
e 0, 1, 2 oder 3,
f 0, 1 oder 2
und die Summe von e+f 0, 1, 2 oder 3 ist,
mit der Maßgabe, daß durchschnittlich mindestens 2 Si-gebundene Wasserstoffatome je Molekül vorliegen, verwendet. Die Organopolysiloxane (2) besitzen vorzugsweise eine durchschnittliche Viskosität von 10 bis 1 000 mPa·s bei 25°C.

Als Organopolysiloxane (3), die aliphatische Kohlenstoff-Kohlenstoff-Mehrfachbindungen und Si-gebundene Wasserstoffatome aufweisen und anstelle von Organopolysiloxanen (1) und (2) verwendet werden können, werden vorzugsweise solche aus Einheiten der Formel

$$R^7_kSiO_{\frac{4-k}{2}},$$

$$R^7_lR^8SiO_{\frac{3-l}{2}}$$

und

$$R^7_pHSiO_{\frac{3-p}{2}},$$

wobei $R^7$ und $R^8$ die oben dafür angegebene Bedeutung haben,
k 0, 1, 2 oder 3,
l 0, 1 oder 2,
p 0, 1 oder 2, ist
mit der Maßgabe, daß je Molekül durchschnittlich mindestens 2 Reste $R^8$ und durchschnittlich mindestens 2 Si-gebundene Wasserstoffatome vorliegen, verwendet.

Beispiele für Organopolysiloxane (3) sind solche aus $SiO_{4/2}$-, $R^7_3SiO_{1/2}$-, $R^7_2R^8SiO_{1/2}$- und $R^7_2HSiO_{1/2}$-Einheiten, sogenannte MQ-Harze, wobei diese Harze T-Einheiten ($R^7SiO_{3/2}$) und D-Einheiten ($R^7_2SiO$) enthalten können.

Die Organopolysiloxane (3) besitzen vorzugsweise eine durchschnittliche Viskosität von 100 bis 100 000 mPa·s bei 25°C bzw. sind Feststoffe mit Molekulargewichten von 5 000 bis 50 000 g/mol.

Die erfindungsgemäßen Katalysatoren werden vorzugsweise in Mengen von 1 bis 1 000 Gew.-ppm (Gewichtsteilen je Million Gewichtsteilen), bevorzugt 10 bis 100 Gew.-ppm, jeweils berechnet als elementares Übergangsmetall Pt, Pd, Ru, Rh, Os oder Ir und bezogen auf das Gesamtgewicht der Organopolysiloxane (1) und (2) bzw. auf das Gesamtgewicht der Organopolysiloxane (3), eingesetzt.

Obwohl nicht bevorzugt, können bei den vernetzbaren Organopolysiloxanzusammensetzungen Inhibitoren mitverwendet werden. Beispiele für Inhibitoren sind 1,3-Divinyl-1,1,3,3-tetramethyldisiloxan, Benzotriazol, Dialkylformamide, Alkylthioharnstoffe, Methylethylketoxim, organische oder siliciumorganische Verbindungen mit einem Siedepunkt von mindestens 25°C bei 1012 mbar (abs.) und mindestens einer aliphatischen Dreifachbindung gemäß US-A 3,445,420, wie 1-Ethinylcyclohexan-1-ol, 2-Methyl-3-butin-2-ol, 3-Methyl-1-pentin-3-ol, 2,5-Dimethyl-3-hexin-2,5-diol und 3,5-Dimethyl-1-hexin-3-ol, Inhibitoren gemäß US-A 2,476,166, wie eine Mischung aus Diallylmaleinat und Vinylacetat, und Inhibitoren gemäß US 4,504,645, wie Maleinsäuremonoester.

Die erfindungsgemäßen Katalysatoren können weiterhin bei allen Verfahren zur Umsetzung von Si-gebundenen Wasserstoffatome aufweisenden Organosiliciumverbindungen mit aliphatischen Mehrfachbindungen aufweisenden organischen Verbindungen eingesetzt werden, bei denen auch bisher die Anlagerung von Si-gebundenen Wasserstoff an aliphatische Mehrfachbindung fördernde Katalysatoren eingesetzt werden konnten.

Gegenstand der Erfindung ist daher ein Verfahren zur Umsetzung von Si-gebundenen Wasserstoffatome aufweisenden Organosiliciumverbindungen mit aliphatischen Mehrfachbindungen aufweisenden organischen Verbindungen in Gegenwart von Katalysatoren gemäß Formel (I).

Unter organischen Verbindungen mit aliphatischen Mehrfachbindungen sind auch organische Verbindungen mit cycloaliphatischen Mehrfachbindungen zu verstehen.

Beispiele für Si-gebundene Wasserstoffatome aufweisende Organosiliciumverbindungen sind Silane mit einem Si-gebundenen Wasserstoffatom je Molekül, wie Trichlorsilan, Dimethylchlorsilan, Dimethylethoxysilan, Methyldiethoxysilan, Methyldichlorsilan und Triethoxysilan, und Organopolysiloxane mit mindestens einem Si-gebundenen Wasserstoffatom je Molekül, wie $\alpha,\omega$-Dihydrogen[dimethylpolysiloxan], Tetramethyldisiloxan, Tetramethylcyclotetrasiloxan, Mischpolymerisate aus Trimethylsiloxan- und Methylhydrogensiloxaneinheiten, Mischpolymerisate aus Trimethylsiloxan-, Dimethylsiloxan- und Methylhydrogensiloxaneinheiten und Trimethylsiloxyhydrogensilan.

Beispiele für aliphatische Mehrfachbindungen aufweisende organische Verbindungen sind Verbindungen mit aliphatischer Kohlenstoff-Kohlenstoff-Doppelbindung, wie Styrol, Allylglycidether, Allylcyanid, Allylacetat, Allylbernsteinsäureanhydrid, Glycolmonoallylether, Allylmethacrylat, Allylamin und Cyclohexen und Verbindungen mit aliphatischer Kohlenstoff-Kohlenstoff-Dreifachbindung, wie Acetylen und Butinol.

Herstellung der Triazene (im wäßrigen System):

0,25 mol des jeweils in Tabelle 1 genannten Anilin-Derivats wurden in 200 ml 10 %iger wäßriger Salzsäure gelöst, dann mit 1 g Aktivkohle 5 Minuten gerührt, und filtriert. Das Filtrat wurde bei 0°C unter Lichtausschluß mit einer Lösung von 17,25 g (0,25 mol) Natriumnitrit in 30 g Wasser versetzt. Nach 15 Minuten wurde bei 0°C 1 mol des jeweils in Tabelle 1 genannten Alkylamins zugetropft und bei Raumtemperatur 2 Stunden gerührt. Nach Zugabe von organischem Lösungsmittel wurde mit 3 x 50 ml Wasser (bzw. bei Bedarf mit verdünnter Essigsäure) ausgeschüttelt und die organische Phase über Natriumsulfat getrocknet. Nach der Filtration wurde bei Raumtemperatur im Rotationsverdampfer unter reduziertem Druck eingeengt. Mögliche Anteile von Pentazdiennebenprodukten können in methanolischer Lösung bei -65°C als Feststoff abgetrennt werden. Es wurden die in Tabelle 1 genannten Produkte mit Ausbeuten zwischen 60% und 80% erhalten.

Herstellung der Triazene (im organischen System):

0,2 mol des jeweils in Tabelle 1 genannten Anilin-Derivats wurden in 100 ml Aceton gelöst, dann mit 1 g Aktivkohle 5 Minuten gerührt und filtriert. Das Filtrat wurde bei -5°C unter Licht- und Feuchtigkeitsausschluß mit 24,59 g (0,25 mol) wasserfreier Schwefelsäure versetzt und 20 Minuten gerührt. Dann wurden 20,62 g (0,2 mol) n-Butylnitrit zugetropft und weitere 2 Stunden bei 0°C gerührt. Schließlich wurden 0,5 mol des jeweils in Tabelle 1 genannten Alkylamins zugetropft und nochmals 2 Stunden gerührt. Die organische Phase wurde dreimal mit 50 ml wäßriger Salzsäure ausgeschüttelt, über Natriumsulfat getrocknet und am Rotationsverdampfer unter reduziertem Druck bis zur Gewichtskonstanz eingeengt. Es wurden die in Tabelle 1 genannten Produkte in Ausbeuten zwischen 70% und 80% erhalten.

Tabelle 1:

| Anilin-Derivat | Alkylamin | Triazen |
|---|---|---|
| Anilin | n-Hexylamin | 1-Phenyl-3-n-hexyl-1-triazen |
| p-Nitroanilin | n-Octylamin | 1-[4-Nitrophenyl]-3-n-octyl-1-triazen |
| p-Cyanoanilin | n-Hexylamin | 1-[4-Cyanophenyl]-3-n-hexyl-1-triazen |

Herstellung der Pentazdiene:

20 mmol des jeweils in Tabelle 2 genannten Anilin-Derivats wurden in 40 ml 10 %iger wäßriger Salzsäure (109 mmol) gelöst, mit 0,5 g Aktivkohle 5 Minuten gerührt und filtriert. Das Filtrat wurde bei -5°C unter Lichtausschluß mit einer Lösung von 1,38 g (20 mmol) Natriumnitrit in 10 ml Wasser versetzt, und nach 15 Minuten wurde bei 0°C 10 ml einer 25 %igen Ammoniaklösung (147 mmol) zudosiert. Bei Raumtemperatur wurde eine Stunde gerührt. Nach Zugabe von 100 ml organischem Lösungsmittel wurde dreimal mit 50 ml Wasser (bzw. bei Bedarf mit verdünnter Essigsäure) ausgeschüttelt und die organische Phase über Natriumsulfat getrocknet. Die Pentazdiene wurden ohne Aufreinigung (Explosionsgefahr) mit den Übergangsmetallen umgesetzt.

Tabelle 2

| Anilin-Derivat | Produkt |
|---|---|
| Anilin | 1,5-Diphenyl-1,4-pentazdien |
| p-Brom-Anilin | 1,5-Di[4-bromphenyl]-1,4-pentazdien |
| p-Methoxy-Anilin | 1,5-Di[4-methoxyphenyl]-1,4-pentazdien |

Beispiel 1:

2,5 g (12,1 mmol) 1-Phenyl-3-n-hexyl-1-triazen, dessen Herstellung oben beschrieben wurde, wurden unter Lichtausschluß mit 20 ml n-Hexan bei -10°C vorgelegt. Unter Stickstoffatmosphäre wurden langsam 8 ml einer 1,6 molaren Lösung von n-Butyllithium (12,8 mmol) in Hexan zudosiert. Nach 20 Minuten wurde eine Lösung von 1,02 g (3,02 mmol) $PtCl_4$ in 50 ml Tetrahydrofuran zugetropft, und 24 Stunden bei Raumtemperatur gerührt. Die Lösung wurde bei Raumtemperatur und bei reduziertem Druck einrotiert, der Rückstand mit 50 ml n-Hexan aufgenommen, filtriert, und das Filtrat abermals bei Raumtemperatur einrotiert. Es wurden 2,44 g (80 % Ausbeute) des Tetrakis(1-phenyl-3-n-hexyl-1-triazenido)platinkomplexes erhalten.

Beispiel 2:

Die Arbeitsweise von Beispiel 1 wurde wiederholt mit der Abänderung, daß 6,05 mmol anstelle 12,1 mmol 1-Phenyl-3-n-hexyl-1-triazen, 6,4 mmol anstelle 12,8 mmol n-Butyllithium und 3,02 mmol 1,5-Cycclooctadien·$PtCl_2$ anstelle 3,02 mmol $PtCl_4$ eingesetzt wurden. Es wurde der 1,5-Cyclooctadien-bis (1-phenyl-3-n-hexyl-1-triazenido)platinkomplex erhalten.

Beispiel 3:

55,69 g (0,2 mol) 1-[4-Nitrophenyl]-3-n-octyl-1-triazen, dessen Herstellung oben beschrieben wurde, wurde unter Licht- und Feuchtigkeitsausschluß in 300 ml Toluol vorgelegt und mit 20,24 g (0,2 mol) trockenem Triethylamin versetzt. Nach 10 Minuten wurde eine Lösung von 16,84 g (0,05 mol) $PtCl_4$ in 40 ml Tetrahydrofuran zugetropft und 24 Stunden bei Raumtemperatur (bzw. 8 Stunden bei 40°C) gerührt. Die Lösung wurde bei Raumtemperatur und reduziertem Druck auf die Hälfte eingeengt, auf -10°C gekühlt, filtriert und schließlich bis zur Massenkonstanz einrotiert. Es wurden 54,07 g (83 % Ausbeute) des Tetrakis[1-(4-nitrophenyl)-3-n-octyl-1-triazenido]platinkomplexes erhalten.

Beispiel 4:

Die Arbeitsweise von Beispiel 3 wurde wiederholt mit der Abänderung, daß 0,2 mol 1-[4-Cyanophenyl]-3-n-hexyl-1-triazen, dessen Herstellung oben beschrieben wurde, anstelle von 0,2 mol 1-[4-Nitrophenyl]-3-n-octyl-1-triazen eingesetzt wird. Es wurde der Tetrakis[1-(4-cyanophenyl)-3-n-hexyl-1-triazenido]platinkomplex erhalten.

Beispiel 5:

Die Arbeitsweise von Beispiel 3 wurde wiederholt mit der Abänderung, daß 0,1 mol anstelle von 0,2 mol 1-(4-Nitrophenyl)-3-n-octyl-1-triazen, 0,1 mol anstelle von 0,2 mol Triethylamin und 0,05 mol 1,5-Cyclooctadien·$PtCl_2$ anstelle von 0,05 mol $PtCl_4$ eingesetzt wurden. Es wurde der 1,5-Cyclooctadien-bis[1-(4-nitrophenyl)-3-n-octyl-1-triazenido]platinkomplex erhalten.

Beispiel 6:

4,62 g (22,5 mmol) 1-Phenyl-3-n-hexyl-1-triazen, dessen Herstellung oben beschrieben wurde, wurden unter Lichtausschluß in 20 ml Toluol bei -10°C vorgelegt. Unter Stickstoffatmosphäre wurden langsam 15,6 ml einer 1,6 molaren Lösung von n-Butyllithium (25 mmol) in Hexan zudosiert. Nach 20 Minuten wurde eine Lösung von 2 g $PdCl_2$ in 150 ml Tetrahydrofuran zugetropft und es wurde 48 Stunden bei Raumtemperatur gerührt. Die Lösung wurde bei Raumtemperatur unter reduziertem Druck einrotiert, der Rückstand mit 100 ml n-Hexan bei -45°C aufgeschlämmt, filtriert, und das Filtrat wurde wieder bei Raumtemperatur einrotiert. Es wurden 4,17 g (72 % Ausbeute) des Bis(1-phenyl-3-n-hexyl-1-triazenido)palladiumkomplexes erhalten.

Beispiel 7:

1,06 g (5,16 mmol) 1-Phenyl-3-n-hexyl-1-triazen, dessen Herstellung oben beschrieben wurde, wurden unter Lichtausschluß in 20 ml Toluol bei -10°C vorgelegt. Unter Stickstoffatmosphäre wurden langsam 3,75 ml einer 1,6 molaren Lösung von n-Butyllithium (6 mmol) in Hexan zudosiert. Nach 20 Minuten wurde eine Lösung von 2,47 g (2,58 mmol) Tris(triphenylphosphin)ruthenium(II)chlorid in 150 ml Tetrahydrofuran zugetropft, und es wurde 48 Stunden bei Raumtemperatur gerührt. Die Lösung wurde bei Raumtemperatur unter reduziertem Druck einrotiert, der Rückstand mit 100 ml n-Hexan bei -45°C aufgeschlämmt, filtriert, und das Filtrat wurde wieder bei Raumtemperatur einrotiert. Es wurden 2,27 g (68% Ausbeute) des Tris(triphenylphosphin)bis(1-phenyl-3-n-hexyl-1-triazenido)rutheniumkomplexes erhalten.

Beispiel 8:

Die Arbeitsweise von Beispiel 7 wird wiederholt mit der Abänderung, daß 2,39 g Tris(triphenylphosphin)rhodium(I) chlorid anstelle von 2,47 g Tris(triphenylphosphin)ruthenium(II)chlorid, 0,53 g (2,58 mmol) anstelle von 1,06 g (5,16 mmol) 1-Phenyl-3-n-hexyl-1-triazen und 1,88 ml (3 mmol) anstelle von 3,75 ml (6 mmol) n-Butyllithium eingesetzt wurden. Es wurde der Tris (triphenylphosphin)1-phenyl-3-n-hexyl-1-triazenidorhodiumkomplex erhalten.

Beispiel 9:

Zu 3 g (7,4 mmol) 1,5-Di(4-bromphenyl)-1,4-pentazdien, dessen Herstellung oben beschrieben wurde, gelöst in 200 ml Toluol, wurden unter Lichtausschluß und bei 0°C unter Stickstoffatmosphäre langsam 6 ml einer 1,6 molaren Lösung von n-Butyllithium (9,6 mmol) in Hexan zudosiert. Nach 20 Minuten wurde eine Lösung von 0,623 g (1,85 mmol) $PtCl_4$ in 50 ml Tetrahydrofuran zugetropft, und es wurde 24 Stunden bei Raumtemperatur gerührt. Die Lösung wurde bei Raumtemperatur unter reduziertem Druck einrotiert, in 100 ml Toluol gelöst, auf -45°C gekühlt, filtriert und wieder bei Raumtemperatur einrotiert. Es wurden 2,68 g (80% Ausbeute) des Tetrakis[1,5-di(4-bromphenyl)-1,4-pentazdienido] platinkomplexes erhalten.

Beispiel 10:

Die Arbeitsweise von Beispiel 9 wurde wiederholt mit der Abänderung, daß 3,7 mmol 1,5-Cyclooctadien·$PtCl_2$ anstelle von 1,85 mmol $PtCl_4$ eingesetzt wurden. Es wurde der 1,5-Cyclooctadien-bis[1,5-di(4-bromphenyl)-1,4-pentazdienido]platinkomplex erhalten.

Beispiel 11:

6,41 g (22,5 mmol) 1,5-Di(4-methoxyphenyl)-1,4-pentazdien, dessen Herstellung oben beschrieben wurde, wurden unter Lichtausschluß in 20 ml Toluol bei -10°C vorgelegt. Unter Stickstoffatmosphäre wurden langsam 15,6 ml einer 1,6 molaren Lösung von n-Butyllithium (25 mmol) in Hexan zudosiert. Nach 20 Minuten wurde eine Lösung von 2 g (11,25 mmol) $PdCl_2$ in 150 ml Tetrahydrofuran zugetropft und es wurde 48 Stunden bei Raumtemperatur gerührt. Die Lösung wurde bei Raumtemperatur unter reduziertem Druck einrotiert, der Rückstand mit 100 ml n-Hexan bei -45°C aufgeschlämmt, filtriert, und das Filtrat wurde wieder bei Raumtemperatur einrotiert. Es wurden 5,46 g (72 % Ausbeute) des Bis[1,5-di(4-methoxyphenyl)-1,4-pentazdienido]palladiumkomplexes erhalten.

Beispiel 12:

2 mg ($1{,}9 \times 10^{-6}$ mol) Tetrakis(1-phenyl-3-n-hexyl-1-triazenido)platinkomplex, dessen Herstellung in Beispiel 1 beschrieben wurde, wurden in 0,1 ml Toluol gelöst und dann zu 7,46 g $\alpha,\omega$-Divinyldimethylpolysiloxan mit einer Viskosität von 500 mPa·s bei 25°C gegeben. Das Lösungsmittel wurde bei Raumtemperatur unter reduziertem Druck entfernt.

Zur verbleibenden Reaktionsmischung wurden 0,187 g eines Mischpolymerisats aus Trimethylsiloxan- und Methylhydrogensiloxaneinheiten mit einer Viskosität von 33 mPa·s bei 25°C, das 1,12 Gew.-% Si-gebundenen Wasserstoff enthält, gegeben, so daß die Mischung 50 Gew.-ppm Platin, berechnet als Element, enthielt. Die gesamte Mischung war bei Raumtemperatur und unter Lichtausschluß mindestens 6 Wochen stabil. Nach 5 Minuten Bestrahlen mit Ultraviolettlicht (UVA = 56 mW/$cm^2$, UVB = 12 mW/$cm^2$) wurde eine komplette Vernetzung der Masse (die extrahierbaren Anteile, d.h. die nicht vernetzten Anteile, sind weniger als 5 Gew.-%) erzielt. Es wurde ein transparentes, in organischen Lösungsmitteln unlösliches Produkt erhalten.

Beispiel 13:

Die Arbeitsweise von Beispiel 12 wurde wiederholt mit der Abänderung, daß nach 8 Minuten Erwärmen bei 80°C eine komplette Vernetzung der Masse (die extrahierbaren Anteile, d.h. die nicht vernetzten Anteile, sind weniger als 5 Gew.-%) erzielt wurde. Es wurde ein transparentes, in organischen Lösungsmitteln unlösliches Produkt erhalten.

Beispiel 14:

Die Arbeitsweise von Beispiel 12 wurde wiederholt mit der Abänderung, daß nach 4,5 Minuten Erwärmen bei 100°C eine komplette Vernetzung der Masse (die extrahierbaren Anteile, d.h. die nicht vernetzten Anteile, sind weniger als 5 Gew.-%) erzielt wurde. Es wurde ein transparentes, in organischen Lösungsmitteln unlösliches Produkt erhalten.

Beispiel 15:

Die Arbeitsweise von Beispiel 12 wurde wiederholt mit der Abänderung, daß nach 2,3 Minuten Erwärmen bei 120°C eine komplette Vernetzung der Masse (die extrahierbaren Anteile, d.h. die nicht vernetzten Anteile, sind weniger als 5 Gew.-%) erzielt wurde. Es wurde ein transparentes, in organischen Lösungsmitteln unlösliches Produkt erhalten.

Beispiel 16:

Die Arbeitsweise von Beispiel 12 wurde wiederholt mit der Abänderung, daß ein $\alpha,\omega$-Divinyldimethylpolysiloxan mit einer Viskosität von 1 000 mPa·s bei 25°C anstelle von 500 mPa·s bei 25°C eingesetzt wurde. Nach 5 Minuten Bestrahlen mit Ultraviolettlicht (UVA = 56 mW/$cm^2$, UVB = 12 mW/$cm^2$) wurde eine komplette Vernetzung der Masse (die extrahierbaren Anteile, d.h. die nicht vernetzten Anteile, sind weniger als 5 Gew.-%) erzielt. Es wurde ein transparentes, in organischen Lösungsmitteln unlösliches Produkt erhalten.

Beispiel 17:

Die Arbeitsweise von Beispiel 12 wurde wiederholt mit der Abänderung, daß 4,9 mg ($3{,}7 \times 10^{-6}$ mol) Tris(triphenylphosphin) bis(1-phenyl-3-n-hexyl-1-triazenido)rutheniumkomplex, dessen Herstellung in Beispiel 7 beschrieben wurde, anstelle von 2 mg Tetrakis(1-phenyl-3-n-hexyl-1-triazenido)platinkomplex eingesetzt wurden. Die Mischung enthielt 50 Gew.-ppm Ruthenium, berechnet als Element. Die gesamte Mischung war bei Raumtemperatur und unter Lichtausschluß mindestens 9 Wochen stabil. Nach 31 Minuten Erwärmen bei 180°C wurde eine komplette Vernetzung der Masse (die extrahierbaren Anteile, d.h. die nicht vernetzten Anteile, sind weniger als 5 Gew.-%) erzielt. Es wurde eine klare spröde Masse erhalten.

Beispiel 18:

Die Arbeitsweise von Beispiel 17 wurde wiederholt mit der Abänderung, daß ein $\alpha,\omega$-Divinyldimethylpolysiloxan mit einer Viskosität von 1 000 mPa·s bei 25°C anstelle von 500 mPa·s bei 25°C eingesetzt wurde. Die gesamte Mischung war bei Raumtemperatur und unter Lichtausschluß mindestens 9 Wochen stabil. Nach 31 Minuten Erwärmen bei 180°C wurde eine komplette Vernetzung der Masse (die extrahierbaren Anteile, d.h. die nicht vernetzten Anteile, sind weniger als 5 Gew.-%) erzielt. Es wurde eine klare spröde Masse erhalten.

Beispiel 19:

Die Arbeitsweise von Beispiel 12 wurde wiederholt mit der Abänderung, daß 1,85 mg ($3{,}6 \times 10^{-6}$ mol) Bis(1-phenyl-3-n-hexyl-1-triazenido)palladiumkomplex, dessen Herstellung in Beispiel 6 beschrieben wurde, anstelle von 2 mg Tetrakis(1-phenyl-3-n-hexyl-1-triazenido)platinkomplex eingesetzt wurde. Die Mischung enthielt 50 Gew.-ppm Palladium, berechnet als Element. Die gesamte Mischung war bei Raumtemperatur und unter Lichtausschluß mindestens 9

Wochen stabil. Nach 13 Minuten Erwärmen bei 170°C wurde eine komplette Vernetzung der Masse (die extrahierbaren Anteile, d.h. die nicht vernetzten Anteile, sind weniger als 5 Gew.-%) erzielt. Es wurde eine klare spröde Masse erhalten.

Beispiel 20:

Die Arbeitsweise von Beispiel 19 wurde wiederholt mit der Abänderung, daß ein α,ω-Divinyldimethylpolysiloxan mit einer Viskosität von 1 000 mPa·s bei 25°C anstelle von 500 mPa·s bei 25°C eingesetzt wurde. Die gesamte Mischung war bei Raumtemperatur und unter Lichtausschluß mindestens 9 Wochen stabil. Nach 13 Minuten Erwärmen bei 170°C wurde eine komplette Vernetzung der Masse (die extrahierbaren Anteile, d.h. die nicht vernetzten Anteile, sind weniger als 5 Gew.-%) erzielt. Es wurde eine klare spröde Masse erhalten.

Beispiel 21:

In 0,5 g eines Organopolysiloxanharzes aus $SiO_2$-, Trimethylsiloxan-, Dimethylvinylsiloxan- und Methylphenylsiloxaneinheiten mit einer Viskosität von 1 600 mPa·s bei 25°C, das 7,6 Gew.-% Si-gebundene Vinylgruppen enthält, wurde eine Lösung von 0,636 mg ($1{,}0x10^{-6}$ mol) Tetrakis(1-phenyl-3-n-hexyl-1-triazenido)platinkomplex, dessen Herstellung in Beispiel 1 beschrieben wurde, in 0,1 ml Toluol gelöst. Das Lösungsmittel wurde bei Raumtemperatur unter reduziertem Druck entfernt, und zur verbleibenden Reaktionsmischung wurden 5 g eines Organopolysiloxanharzes aus $SiO_2$-, Trimethylsiloxan-, Dimethylhydrogensiloxan- und Methylphenylsiloxaneinheiten mit einer Viskosität von 2 000 mPa·s bei 25°C, das 0,2 Gew.-% Si-gebundenen Wasserstoff enthält, gegeben, so daß die Mischung 21 Gew.-ppm Platin, berechnet als Element, enthielt. Die gesamte Mischung war bei Raumtemperatur und unter Lichtausschluß mindestens 6 Wochen stabil. Bei guter Durchmischung wurde nach 15 Minuten Erwärmen bei 170°C eine komplette Vernetzung der Masse erzielt. Es wurde eine klare unlösliche Substanz erhalten.

Beispiel 22:

Die Arbeitsweise von Beispiel 21 wurde wiederholt mit der Abänderung, daß 1,41 mg ($1{,}0x10^{-6}$ mol) Tris (triphenylphosphin) bis (1-phenyl-3-n-hexyl-1-triazenido)rutheniumkomplex, dessen Herstellung in Beispiel 7 beschrieben wurde, anstelle von 0,636 mg Tetrakis (1-phenyl-3-n-hexyl-1-triazenido) platinkomplex, eingesetzt wurden. Die Mischung enthielt 20 Gew.-ppm Ruthenium, berechnet als Element. Die gesamte Mischung war bei Raumtemperatur und unter Lichtausschluß mindestens 9 Wochen stabil. Bei guter Durchmischung wurde nach 30 Minuten Erwärmen bei 180°C eine komplette Vernetzung der Masse erzielt. Es wurde eine klare unlösliche Substanz erhalten.

Beispiel 23:

Die Arbeitsweise von Beispiel 21 wurde wiederholt mit der Abänderung, daß 0,53 mg ($1{,}0x10^{-6}$ mol) Bis(1-phenyl-3-n-hexyl-1-triazenido)palladiumkomplex, dessen Herstellung in Beispiel 6 beschrieben wurde, anstelle von 0,636 mg Tetrakis(1-phenyl-3-n-hexyl-1-triazenido)platinkomplex, eingesetzt wurden. Die Mischung enthielt 20 Gew.-ppm Palladium, berechnet als Element. Die gesamte Mischung war bei Raumtemperatur und unter Lichtausschluß mindestens 9 Wochen stabil. Bei guter Durchmischung wurde nach 30 Minuten Erwärmen bei 170°C eine komplette Vernetzung der Masse erzielt. Es wurde eine klare unlösliche Substanz erhalten.

Beispiel 24:

4 g eines Organopolysiloxanharzes der Formel $(SiO_2)_{610}$ $(Me_3SiO_{1/2})_{232}(EtO_{1/2})_{414}(HMe_2SiO_{1/2})_{156}(ViMe_2SiO_{1/2})$ 100 wurden in 16 g Toluol bei Raumtemperatur gelöst, filtriert und unter Rühren wurden 5,19 mg Tetrakis(1-phenyl-3-n-hexyl-1-triazenido)platinkomplex zugemischt, so daß die Mischung 50 Gew.-ppm Platin, berechnet als Element, enthielt. Nach 10 Stunden Erwärmen auf 60°C wurde eine komplette Vernetzung der Masse erzielt. Es wurde ein steifes, leicht gelb gefärbtes Gel erhalten.

Beispiel 25:

3,55 mg ($1{,}96 \cdot 10^{-6}$ mol) Tetrakis[1,5-di(4-bromphenyl-1,4-pentazdienido]platinkomplex, dessen Herstellung in Beispiel 9 beschrieben wurde, wurden in 0,1 ml Toluol gelöst und dann zu 7,46 g α,ω-Divinyldimethylpolysiloxan mit einer Viskosität von 500 mPa·s bei 25°C gegeben. Das Lösungsmittel wurde bei Raumtemperatur unter reduziertem Druck entfernt. Zur verbleibenden Reaktionsmischung wurden 0,187 g eines Mischpolymerisats aus Trimethylsiloxan- und Methylhydrogensiloxaneinheiten mit einer Viskosität von 33 mPa·s bei 25°C, das 1,12 Gew.-% Si-gebundenen Wasserstoff enthält, gegeben, so daß die Mischung 50 Gew.-ppm Platin, berechnet als Element, enthielt. Die gesamte Mischung war bei Raumtemperatur und unter Lichtausschluß mindestens 6 Wochen stabil. Nach 3,5 Minuten Erwärmen bei 100°C wurde eine komplette Vernetzung der Masse (die extrahierbaren Anteile, d.h. die nicht vernetzten Anteile, sind weniger als 5 Gew.-%) erzielt. Es wurde ein transparentes in organischen Lösungsmitteln unlösliches Produkt erhalten.

Beispiel 26:

Die Arbeitsweise von Beispiel 25 wurde wiederholt mit der Abänderung, daß die Mischung durch Bestrahlung mit Ultraviolettlicht (UVA = 56 $mW/cm^2$, UVB = 12 $mW/cm^2$) anstelle durch Erhitzen bei 100°C innerhalb von 3 Minuten vernetzt wurde.

Beispiel 27:

Die Arbeitsweise von Beispiel 25 wurde wiederholt mit der Abänderung, daß 6,56 mg Bis[1,5-di(4-methoxyphenyl-1,4-pentazdienido]palladiumkomplex, dessen Herstellung in Beispiel 11 beschrieben wurde, anstelle von 3,55 mg Tetrakis[1,5-di(4-bromphenyl-1,4-pentazdienido]platinkomplex eingesetzt wurden. Die Mischung enthielt 100 Gew.-ppm Palladium, berechnet als Element. Die gesamte Mischung war bei Raumtemperatur und unter Lichtausschluß mindestens 6 Wochen stabil. Nach 22 Minuten Erwärmen bei 150°C wurde eine komplette Vernetzung der Masse (die extrahierbaren Anteile, d.h. die nicht vernetzten Anteile, sind weniger als 5 Gew.-%) erzielt. Es wurde ein transparentes in organischen Lösungsmitteln unlösliches Produkt erhalten.

Beispiel 28:

140 g (0,85 mol) Hydrogentriethoxysilan wurden mit 100 g (0,88 mol) Allylglycidether gemischt und 10 g dieser Mischung wurden mit 100 mg ($9,5 \times 10^{-5}$ mol) Tetrakis(1-phenyl-3-n-hexyl-1-triazenido)platinkomplex in einem Reaktionsgefäß bei 95°C unter Normaldruck vorgelegt. Beim Zutropfen der restlichen oben genannten Mischung innerhalb von 80 Minuten, stieg die Temperatur bis auf 160°C an. Nach vollständiger Zugabe wurde die Reaktionsmischung noch 30 Minuten bei 150°C gerührt. Nach der Destillation wurde 3-Glycidoxypropyltriethoxysilan als farbloses, flüssiges Produkt in 62,6 % Ausbeute erhalten.

Beispiel 29:

0,8 mg ($7{,}0 \cdot 10^{-7}$mol) Tetrakis(1-phenyl-3-n-hexyl-1-triazenido)platinkomplex wurden in 0,1 ml Toluol gelöst, zu 7,46 g α,ω-Divinyldimethylpolysiloxan mit einer Viskosität von 500 mPa·s bei 25°C gegeben und mit 0,3 g einer 50 %igen Lösung eines Sulfoniumsalzes der Formel

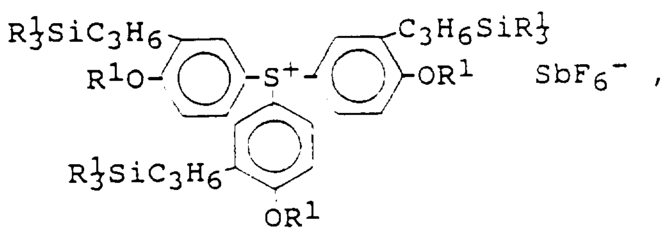

($R^1$ = n-Butylrest)

in Hexan (20 Gew. ppm, bezogen auf die Mischung) versetzt. Die Lösungsmittel wurden bei Raumtemperatur unter reduziertem Druck entfernt, und zur verbleibenden Reaktionsmischung wurden 0,187 g eines Mischpolymerisates aus Trimethylsiloxan- und Methylhydrogensiloxaneinheiten mit einer Viskosität von 33 mPa·s bei 25°C, das 1,12 Gew.-% Si-gebundenen Wasserstoff enthält, gegeben (20 Gew.-ppm, bezogen auf das Gesamtgewicht der Mischung). Die Mischung ist bei Raumtemperatur und unter Lichtausschluß mindestens 6 Wochen stabil. Die komplette Vernetzung (extrahierbare Anteile < 5 Gew.-%) wurde durch UV-Licht (UVA = 56 $mW/cm^2$, UVB = 12 $mW/cm^2$) in 3,2 Minuten erreicht. Es wurde ein transparentes, unlösliches Produkt erhalten.

**Patentansprüche**

1. Verwendung der Übergangsmetallkomplexe der allgemeinen Formel

$$MX_aY_b$$

wobei M Pt, Pd, Rh, Ru, Os oder Ir ist,
X einen Triazen-, Tetrazen-, Tetrazdien- oder Pentazdien-Liganden ausgewählt aus der Gruppe von ANNNR, $ANNNRR^1$, $ANNNA^1$, $ANR^1NNNR^2A^1$, $ANNNNA^1$, $ANNNR^3NNA^1$ und $ANNNNNA^1$ bedeutet, wobei R einen einwertigen, gegebenenfalls substituierten Kohlenwasserstoffrest mit 1 bis 18 Kohlenstoffatomen je Rest oder einen Rest der Formel $-SiR^6_c(OR^6)_{3-c}$,
$R^1$, $R^2$ und $R^3$ gleich oder verschieden sind, ein Wasserstoffatom oder einen einwertigen gegebenenfalls substituierten Kohlenwasserstoffrest mit 1 bis 18 Kohlenstoffatomen je Rest,
und A und $A^1$ gleich oder verschieden sind, einen Rest der Formel

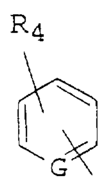
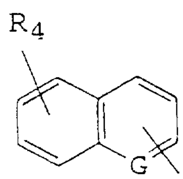
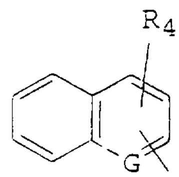
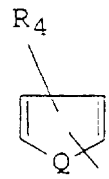
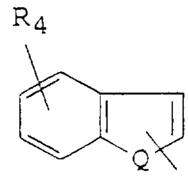

oder

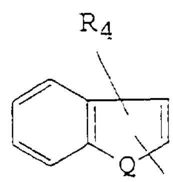

bedeuten, wobei G CH oder N und Q S, O oder NH ist,
$R^4$ einen einwertigen gegebenenfalls substituierten Kohlenwasserstoffrest mit 1 bis 12 Kohlenstoffatomen je Rest oder einen Rest der Formel -F, -Cl, -Br, -I, -H, $-NH_2$, $-NR^6_2$, $-NO_2$, -OH, $-OR^6$, -SH, -CN, -COOH, -COCl, $-CONH_2$, $-COR^6$, -CHO, $-SO_2NHR^6$, $-SO_3H$, $-SO_2Cl$ oder $-R^5-SiR^6_c(OR^6)_{3-c}$,
$R^5$ einen zweiwertigen Kohlenwasserstoffrest mit 1 bis 8 Kohlenstoffatomen je Rest,
$R^6$ gleich oder verschieden ist und einen Alkylrest mit 1 bis 8 Kohlenstoffatomen je Rest und
c 0, 1, 2 oder 3 bedeutet,
Y gleich oder verschieden ist und einen Liganden, ausgewählt aus der Gruppe von Cl, Br, I, $NH_3$, $P(C_2H_5)_3$, $P(C_6H_5)_3$, H, CO, 1,5-Cyclooctadien, Pyridin, Bipyridin, Acetat, Acetylacetonat, Phenylnitril, Ethylendiamin, Acetonitril, 2,5-Norbornadien, Nitrat, Nitrit, $H_2O$, Benzol, Diphenylphosphinoethan und 1,3-Divinyl-1,1,3,3-tetramethyldisiloxan,
a 1, 2, 3 oder 4 und
b 0 oder eine ganze Zahl von 1 bis 6 bedeutet,
als die Anlagerung von Si-gebundenem Wasserstoff an aliphatische Mehrfachbindung fördernde Katalysatoren.

**2.** Übergangsmetallkomplexe der allgemeinen Formel

$$M'X'_aY_b$$

wobei M' Pt, Pd, Rh und Ru ist,
X' einen 1-Triazen-Liganden der allgemeinen Formel
ANNNR' bedeutet, wobei R' einen einwertigen, gegebenenfalls substituierten Kohlenwasserstoffrest mit 1 bis 18 Kohlenstoffatomen je Rest, wobei aromatische Kohlenwasserstoffreste, bei denen der Aromat direkt am Stickstoffatom gebunden ist, ausgeschlossen sind, oder einen Rest der Formel $-SiR^6_c(OR^6)_{3-c}$ bedeutet,
und A einen Rest der Formel

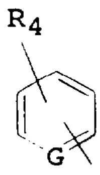 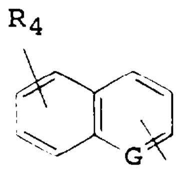 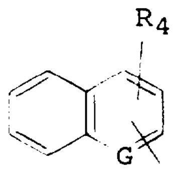 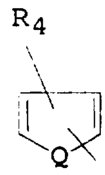 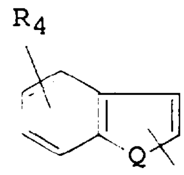

oder

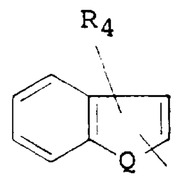

bedeuten, wobei G CH oder N und Q S, O oder NH ist,
$R^4$ einen einwertigen gegebenenfalls substituierten Kohlenwasserstoffrest mit 1 bis 12 Kohlenstoffatomen je Rest oder einen Rest der Formel -F, -Cl, -Br, -I, -H, $-NH_2$, $-NR^6_2$, $-NO_2$, -OH, $-OR^6$, -SH, -CN, -COOH, -COCl, $-CONH_2$, $-COR^6$, -CHO, $-SO_2NHR^6$, $-SO_3H$, $-SO_2Cl$ oder $-R^5-SiR^6_c(OR^6)_{3-c}$,
$R^5$ einen zweiwertigen Kohlenwasserstoffrest mit 1 bis 8 Kohlenstoffatomen je Rest,
$R^6$ gleich oder verschieden ist und einen Alkylrest mit 1 bis 8 Kohlenstoffatomen je Rest und
c 0, 1, 2 oder 3 bedeutet,
Y gleich oder verschieden ist und einen Liganden, ausgewählt aus der Gruppe von Cl, Br, I, $NH_3$, $P(C_2H_5)_3$, $P(C_6H_5)_3$, H, CO, 1,5-Cyclooctadien, Pyridin, Bipyridin, Acetat, Acetylacetonat, Phenylnitril, Ethylendiamin, Acetonitril, 2,5-Norbornadien, Nitrat, Nitrit, $H_2O$, Benzol, Diphenylphosphinoethan und 1,3-Divinyl-1,1,3,3-tetramethyldisiloxan,
a 1, 2, 3 oder 4 und
b 0 oder eine ganze Zahl von 1 bis 6 bedeutet,
mit der Maßgabe, daß Platin-Triazenido-Komplexe der Formel

$$PtZ_2[ANNN(CH_3)]_2,$$

wobei Z Cl oder I bedeutet und A die oben dafür angegebene Bedeutung hat, ausgeschlossen sind.

**3.** Verfahren zur Herstellung der Komplexe nach Anspruch 2, dadurch gekennzeichnet, daß Triazene der Formel ANNNHR' wobei R' einen einwertigen, gegebenenfalls substituierten Kohlenwasserstoffrest mit 1 bis 18 Kohlenstoffatomen je Rest, wobei aromatische Kohlenwasserstoffreste, bei denen der Aromat direkt am Stickstoffatom gebunden ist, ausgeschlossen sind, oder einen Rest der Formel $-SiR^6_c(OR^6)_{3-c}$ bedeutet, und A einen Rest der Formel

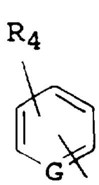 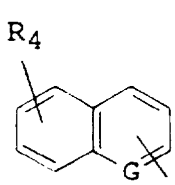 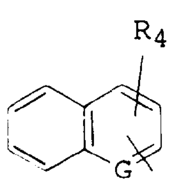 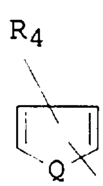 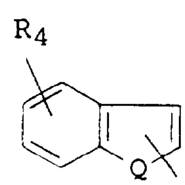

oder

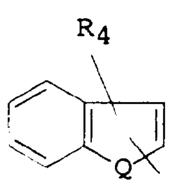

bedeutet, wobei G CH oder N und Q S, O oder NH ist,
$R^4$ einen einwertigen gegebenenfalls substituierten Kohlenwasserstoffrest mit 1 bis 12 Kohlenstoffatomen je Rest oder einen Rest der Formel -F, -Cl, -Br, -I, -H, $-NH_2$, $-NR^6_2$, $-NO_2$, -OH, $-OR^6$, -SH, -CN, -COOH, -COCl, $-CONH_2$, $-COR^6$, -CHO, $-SO_2NHR^6$, $-SO_3H$, $-SO_2Cl$ oder $-R^5-SiR^6_c(OR^6)_{3-c}$,
$R^5$ einen zweiwertigen Kohlenwasserstoffrest mit 1 bis 8 Kohlenstoffatomen je Rest,
$R^6$ gleich oder verschieden ist und einen Alkylrest mit 1 bis 8 Kohlenstoffatomen je Rest und
c 0, 1, 2 oder 3 bedeutet,
in Gegenwart von Basen mit Übergangsmetallverbindungen der Formel

$$M'Y_d$$

wobei M' Pt, Pd, Rh und Ru,
Y gleich oder verschieden ist und einen Liganden, ausgewählt aus der Gruppe von Cl, Br, I, $NH_3$, $P(C_2H_5)_3$, $P(C_6H_5)_3$, H, CO, 1,5-Cyclooctadien, Pyridin, Bipyridin, Acetat, Acetylacetonat, Phenylnitril, Ethylendiamin, Acetonitril, 2,5-Norbornadien, Nitrat, Nitrit, $H_2O$, Benzol, Diphenylphosphinoethan und 1,3-Divinyl-1,1,3,3-tetramethyldisiloxan
und d eine ganze Zahl von 1 bis 8 bedeutet,
umgesetzt wird.

**4.** Verfahren zur Aktivierung der Katalysatoren nach Anspruch 2 durch Erhitzen bei Temperaturen von 50°C bis 250°C und/oder durch Bestrahlen mit Licht und/oder durch Zugabe von Brönsted-Säuren und/oder durch Zugabe von säurebildenden Agentien.

**5.** Vernetzbare Organopolysiloxanzusammensetzungen enthaltend

(1) Organopolysiloxane, die Reste mit aliphatischen Kohlenstoff-Kohlenstoff-Mehrfachbindungen aufweisen,
(2) Organopolysiloxane mit Si-gebundenen Wasserstoffatomen oder anstelle von Organopolysiloxanen (1) und (2)
(3) Organopolysiloxane, die Reste mit aliphatischen Kohlenstoff-Kohlenstoff-Mehrfachbindungen und Si-gebundene Wasserstoffatome aufweisen, und
(4) Katalysatoren nach Anspruch 1.

**6.** Verfahren zur Umsetzung von Si-gebundenen Wasserstoffatome aufweisenden Organosiliciumverbindungen mit aliphatischen Mehrfachbindungen aufweisenden organischen Verbindungen in Gegenwart von Katalysatoren nach Anspruch 1.

## Claims

**1.** Use of the transition metal complexes of the general formula

$$MX_aY_b$$

in which M is Pt, Pd, Rh, Ru, Os or Ir,

X denotes a triazene, tetrazene, tetrazadiene or pentazadiene ligand chosen from the group comprising

ANNNR, $ANNNRR^1$, $ANNNA^1$, $ANR^1NNNR^2A^1$, $ANNNNA^1$, $ANNNR^3NNA^1$ and $ANNNNNA^1$

in which R denotes a monovalent, optionally substituted hydrocarbon radical having 1 to 18 carbon atoms per radical or a radical of the formula $-SiR^6{}_c(OR^6)_{3-c}$,

$R^1$, $R^2$ and $R^3$ are identical or different and denote a hydrogen atom or a monovalent optionally substituted hydrocarbon radical having 1 to 18 carbon atoms per radical,

and A and $A^1$ are identical or different and denote a radical of the formula

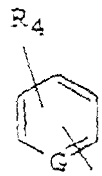 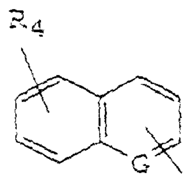 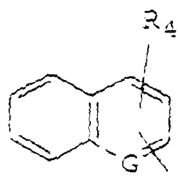 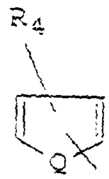 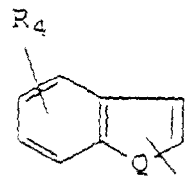

or

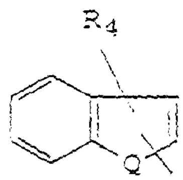

in which G is CH or N and Q is S, O or NH,

$R^4$ denotes a monovalent optionally substituted hydrocarbon radical having 1 to 12 carbon atoms per radical or a radical of the formula -F, -Cl, -Br, -I, -H, $-NH_2$, $-NR^6{}_2$, $-NO_2$, -OH, $-OR^6$, -SH, -CN, -COOH, -COCl, $-CONH_2$, $-COR^6$, -CHO, $-SO_2NHR^6$, $-SO_3H$, $-SO_2Cl$ or $-R^5-SiR^6{}_c(OR^6)_{3-c}$,

$R^5$ denotes a divalent hydrocarbon radical having 1 to 8 carbon atoms per radical,

$R^6$ is identical or different and denotes an alkyl radical having 1 to 8 carbon atoms per radical and

c denotes 0, 1, 2 or 3,

Y is identical or different and denotes a ligand chosen from the group comprising Cl, Br, I, $NH_3$, $P(C_2H_5)_3$, $P(C_6H_5)_3$, H, CO, 1,5-cyclooctadiene, pyridine, bipyridine, acetate, acetylacetonate, phenylnitrile, ethylenediamine, acetonitrile, 2,5-norbornadiene, nitrate, nitrite, $H_2O$, benzene, diphenylphosphinoethane and 1,3-divinyl-1,1,3,3-tetramethyldisiloxane,

a denotes 1, 2, 3 or 4 and

b denotes 0 or an integer from 1 to 6,

as catalysts which promote the addition of Si-bonded hydrogen onto aliphatic multiple bond.

**2.** Transition metal complexes of the general formula

$M'X'_aY_b$

in which M' is Pt, Pd, Rh or Ru,
X' denotes a 1-triazene ligand of the general formula

ANNNR'

in which R' denotes a monovalent, optionally substituted hydrocarbon radical having 1 to 18 carbon atoms per radical, excluding aromatic hydrocarbon radicals in which the aromatic part is bonded directly to the nitrogen atom, or a radical of the formula $-SiR^6{}_c(OR^6)_{3-c}$, and
A denotes a radical of the formula

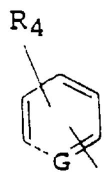 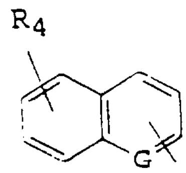 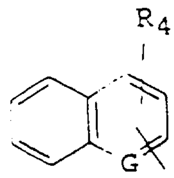 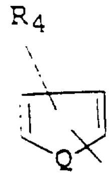 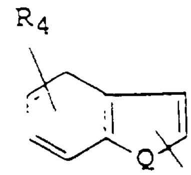

or

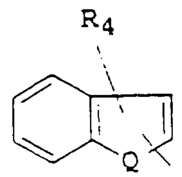

in which G is CH or N and Q is S, O or NH,
$R^4$ denotes a monovalent optionally substituted hydrocarbon radical having 1 to 12 carbon atoms per radical or a radical of the formula -F, -Cl, -Br, -I, -H, $-NH_2$, $-NR^6{}_2$, $-NO_2$, -OH, $-OR^6$, -SH, -CN, -COOH, -COCl, $-CONH_2$, $-COR^6$, -CHO, $-SO_2NHR^6$, $-SO_3H$, $-SO_2Cl$ or $-R^5-SiR^6{}_c(OR^6)_{3-c}$,
$R^5$ denotes a divalent hydrocarbon radical having 1 to 8 carbon atoms per radical,
$R^6$ is identical or different and denotes an alkyl radical having 1 to 8 carbon atoms per radical and
c denotes 0, 1, 2 or 3,
Y is identical or different and denotes a ligand chosen from the group comprising Cl, Br, I, $NH_3$, $P(C_2H_5)_3$, $P(C_6H_5)_3$, H, CO, 1,5-cyclooctadiene, pyridine, bipyridine, acetate, acetylacetonate, phenylnitrile, ethylenediamine, acetonitrile, 2,5-norbornadiene, nitrate, nitrite, $H_2O$, benzene, diphenylphosphinoethane and 1,3-divinyl-1,1,3,3-tetramethyldisiloxane,
a denotes 1, 2, 3 or 4 and
b denotes 0 or an integer from 1 to 6,
with the proviso that platinum-triazenido complexes of the formula

$$PtZ_2[ANNN(CH_3)]_2,$$

in which Z denotes Cl or I and A has the meaning given above for this symbol, are excluded.

**3.** Process for the preparation of the complexes according to Claim 2, characterized in that triazenes of the formula
ANNNHR'

in which R' denotes a monovalent, optionally substituted hydrocarbon radical having 1 to 18 carbon atoms per radical, excluding aromatic hydrocarbon radicals in which the aromatic part is bonded directly to the nitrogen atom, or a radical of the formula $-SiR^6{}_c(OR^6)_{3-c}$, and A denotes a radical of the formula

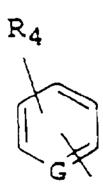 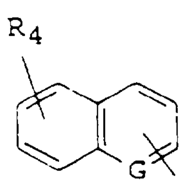 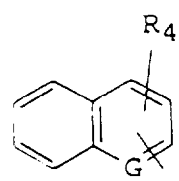 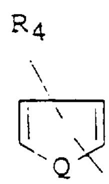 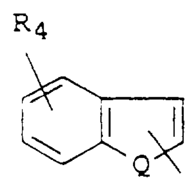

or

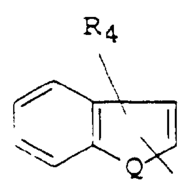

in which G is CH or N and Q is S, O or NH,
$R^4$ denotes a monovalent optionally substituted hydrocarbon radical having 1 to 12 carbon atoms per radical or a radical of the formula -F, -Cl, -Br, -I, -H, $-NH_2$, $-NR^6{}_2$, $-NO_2$, -OH, $-OR^6$, -SH, -CN, -COOH, -COCl, $-CONH_2$,

-$COR^6$, -CHO, -$SO_2NHR^6$, -$SO_3H$, -$SO_2Cl$ or -$R^5$-$SiR^6{}_c(OR^6)_{3-c}$,
$R^5$ denotes a divalent hydrocarbon radical having 1 to 8 carbon atoms per radical,
$R^6$ is identical or different and denotes an alkyl radical having 1 to 8 carbon atoms per radical and
c denotes 0, 1, 2 or 3,
is [sic] reacted with transition metal compounds of the formula

$M'Y_d$

in which M' denotes Pt, Pd, Rh or Ru,
Y is identical or different and denotes a ligand chosen from the group comprising Cl, Br, I, $NH_3$, $P(C_2H_5)_3$, $P(C_6H_5)_3$, H, CO, 1,5-cyclooctadiene, pyridine, bipyridine, acetate, acetylacetonate, phenylnitrile, ethylenediamine, acetonitrile, 2,5-norbornadiene, nitrate, nitrite, $H_2O$, benzene, diphenylphosphinoethane and 1,3-divinyl-1,1,3,3-tetramethyldisiloxane
and d denotes an integer from 1 to 8,
in the presence of bases.

**4.** Process for activation of the catalysts according to Claim 2 by heating at temperatures of 50°C to 250°C and/or by irradiation with light and/or by addition of Brönsted acids and/or by addition of acid-forming agents.

**5.** Crosslinkable organopolysiloxane compositions comprising

(1) organopolysiloxanes which contain radicals with aliphatic carbon-carbon multiple bonds,
(2) organopolysiloxanes with Si-bonded hydrogen atoms or, instead of organopolysiloxanes (1) and (2),
(3) organopolysiloxanes which contain radicals with aliphatic carbon-carbon multiple bonds and Si-bonded hydrogen atoms, and
(4) catalysts according to Claim 1.

**6.** Process for the reaction of organosilicon compounds containing Si-bonded hydrogen atoms with organic compounds containing aliphatic multiple bonds in the presence of catalysts according to Claim 1.

## Revendications

**1.** Utilisation de complexes de métaux de transition de formule générale

$$MX_aY_b,$$

M étant un Pt, un Pd, un Rh, un Ru, un Os ou un Ir,

X signifiant un ligand triazène, tétrazène, tétrazodiène ou pentazodiène sélectionné parmi le groupe des

ANNNR, $ANNNRR^1$, $ANNNA^1$, $ANR^1NNNR^2A^1$, $ANNNNA^1$, $ANNNR^3NNA^1$ et $ANNNNNA^1$,

R signifiant un reste hydrocarboné monovalent, éventuellement substitué, chaque reste ayant de 1 à 18 atomes de carbone ou bien un reste de formule -$SiR_c{}^6\,(OR^6)_{3-c}$,

$R^1$, $R^2$ et $R^3$ étant identiques ou différents et signifiant un atome d'hydrogène ou un reste hydrocarboné monovalent, éventuellement substitué, chaque reste ayant de 1 à 18 atomes de carbone,

et A et $A^1$ étant identiques ou différents et signifiant un reste de formules

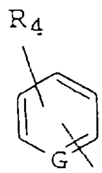 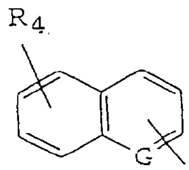 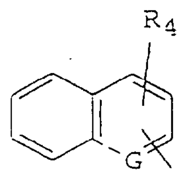 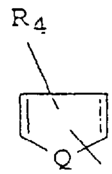 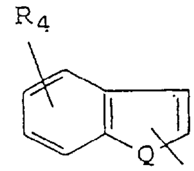

ou

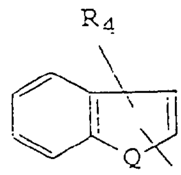

G étant un CH ou un N et Q étant un S, un O ou un NH,

$R^4$ signifiant un reste hydrocarboné monovalent, éventuellement substitué, chaque reste ayant de 1 à 12 atomes de carbone, ou bien un reste de formule -F, -Cl, -Br, -I, -H, $-NH_2$, $-NR_2{}^6$, $-NO_2$, -OH, $-OR^6$, -SH, -CN, -COOH, -COCl, $-CONH_2$, $-COR^6$, -CHO, $-SO_2NHR^6$, $-SO_3H$, $-SO_2Cl$ ou $-R^5-SiR_c{}^6(OR^6)_{3-c}$,

$R^5$ signifiant un reste hydrocarboné bivalent, chaque reste ayant de 1 à 8 atomes de carbone,

$R^6$ étant identique ou différent et signifiant un reste alkyle, chaque reste ayant de 1 à 8 atomes de carbone et c valant 0, 1, 2 ou 3,

Y étant identique ou différent et signifiant un ligand choisi parmi le groupe des Cl, Br, I, $NH_3$, $P(C_2H_5)_3$, $P(C_6H_5)_3$, H, CO, 1,5-cyclooctadiène, pyridine, bipyridine, acétate, acétyle-acétonate, phénylnitrile, éthylènediamine, acétonitrile, 2,5-norbornadiène, nitrate, nitrite, $H_2O$, benzène, diphénylphosphinoéthane et 1,3-divinyl-1,1,3,3-tétraméthyldisiloxane,

a valant 1, 2, 3 ou 4 et

b valant 0 ou étant un nombre entier compris entre 1 et 6,

en tant que catalyseur accélérant la fixation d'hydrogène lié à du Si par addition sur des liaisons multiples aliphatiques.

**2.** Complexes de métal de transition de formule générale

$$M'X'_aY_b,$$

M' étant un Pt, un Pd, un Rh et un Ru,
X' signifiant un ligand 1-triazène de formule générale
ANNNR',
R' signifiant un reste hydrocarboné monovalent, éventuellement substitué, chaque reste ayant de 1 à 18 atomes de carbone, les restes hydrocarbonés aromatiques dont le cycle aromatique est directement lié sur l'atome d'azote, étant exclus, ou bien un reste de formule $-SiR_c{}^6(OR^6)_{3-c}$, et
A signifiant un reste de formule

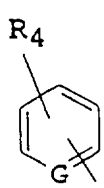
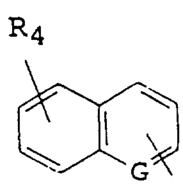
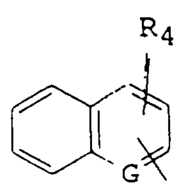
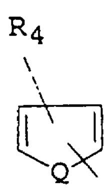
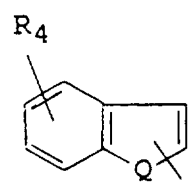

ou

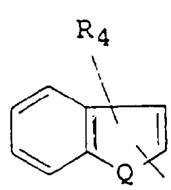

G étant un CH ou un N et Q étant un S, un O ou un NH,

$R^4$ signifiant un reste hydrocarboné monovalent, éventuellement substitué, chaque reste ayant de 1 à 12 atomes de carbone, ou bien un reste de formule -F, -Cl, -Br, -I, -H, $-NH_2$, $-NR_2{}^6$, $-NO_2$, -OH, $-OR^6$, -SH, -CN, -COOH, -COCl, $-CONH_2$, $-COR^6$, -CHO, $-SO_2NHR^6$, $-SO_3H$ , $-SO_2Cl$ ou $-R^5\text{-}SiR_c{}^6(OR^6)_{3-c}$,
$R^5$ signifiant un reste hydrocarboné bivalent, chaque reste ayant de 1 à 8 atomes de carbone,
$R^6$ étant identique ou différent et signifiant un reste alkyle, chaque reste ayant de 1 à 8 atomes de carbone et c valant 0, 1, 2 ou 3,
Y étant identique ou différent et signifiant un ligand choisi parmi le groupe des Cl, Br, I, $NH_3$, $P(C_2H_5)_3$, $P(C_6H_5)_3$, H, CO, 1,5-cyclooctadiène, pyridine, bipyridine, acétate, acétyle-acétonate, phénylnitrile, éthylènediamine, acétonitrile, 2,5-norbornadiène, nitrate, nitrite, $H_2O$, benzène, diphénylphosphinoéthane et 1,3-divinyl-1,1,3,3-tétraméthyldisiloxane,
a valant 1, 2, 3 ou 4 et
b valant 0 ou étant un nombre entier compris entre 1 et 6, avec la condition que les complexes triazénidoplatine de formule

$$PtZ_2[ANNN\,(CH_3)]_2,$$

Z signifiant un Cl ou un I et A ayant la signification donnée à cet effet ci-dessus, sont exclus.

3. Procédé de préparation des complexes selon la revendication 2, caractérisé en ce que des triazènes de formule
ANNNHR',

R' signifiant un reste hydrocarboné monovalent, éventuellement substitué, chaque reste ayant de 1 à 18 atomes de carbone, les restes hydrocarbonés aromatiques dont le noyau aromatique est directement lié à un atome d'azote, étant exclus, ou bien un reste de formule $-SiR_c{}^6(OR^6)_{3-c}$, et
A signifiant un reste de formule

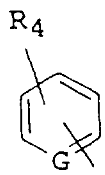
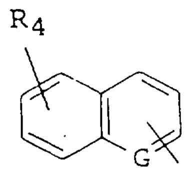
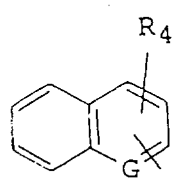
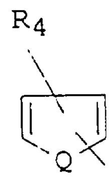
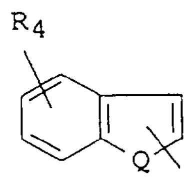

ou

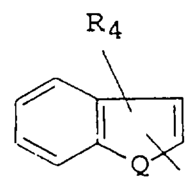

G étant un CH ou un N et Q étant un S, un O ou un NH, $R^4$ signifiant un reste hydrocarboné monovalent, éventuellement substitué, chaque reste ayant de 1 à 12 atomes de carbone, ou bien un reste de formule -F, -Cl, -Br, -I, -H, $-NH_2$, $-NR_2{}^6$, $-NO_2$, -OH, $-OR^6$, -SH, -CN, -COOH, -COCl, $-CONH_2$, $-COR^6$, -CHO, $-SO_2NHR^6$, $-SO_3H$, $-SO_2Cl$ ou $-R^5\text{-}SiR_c{}^6(OR^6)_{3-c}$,
$R^5$ signifiant un reste hydrocarboné bivalent, chaque reste ayant de 1 à 8 atomes de carbone,
$R^6$ étant identique ou différent et signifiant un reste alkyle, chaque reste ayant de 1 à 8 atomes de carbone, et c valant 0, 1, 2 ou 3,
sont mis en réaction en présence de bases avec des composés de métaux de transition de formule
$M'Y_d$
M' signifiant un Pt, un Pd, un Rh ou un Ru,
Y étant identique ou différent et signifiant un ligand choisi parmi le groupe des Cl, Br, I, $NH_3$, $P(C_2H_5)_3$, $P(C_6H_5)_3$, H, CO, 1,5-cyclooctadiène, pyridine, bipyridine, acétate, acétyle-acétonate, phénylnitrile, éthylènediamine, acétonitrile, 2,5-norbornadiène, nitrate, nitrite, $H_2O$, benzène, diphénylphosphinoéthane et 1,3-divinyl-1,1,3,3-tétraméthyldisiloxane,
et d étant un nombre entier compris entre 1 et 8.

4. Procédé d'activation de catalyseurs selon la revendication 2 par chauffage à des températures comprises entre 50°C et 250°C et/ou par irradiation à la lumière et/ou par addition d'acide de Brönsted et/ou par addition d'agents

formant des acides.

**5.** Compositions d'organopolysiloxanes réticulables contenant

(1) des organopolysiloxanes, possédant des restes ayant des liaisons multiples carbone-carbone aliphatiques,
(2) des organopolysiloxanes ayant des atomes d'hydrogène liés à du Si ou bien, à la place de (1) et (2),
(3) des organopolysiloxanes, possédant des restes ayant des liaisons multiples carbone-carbone aliphatiques et des atomes d'hydrogène liés à du Si, et
(4) des catalyseurs selon la revendication 1.

**6.** Procédé de transformation de composés organosiliciés, possédant des atomes d'hydrogène liés à du Si, avec des composés organiques, possédant des liaisons multiples aliphatiques, en présence de catalyseurs selon la revendication 1.